# EUROPEAN PATENT SPECIFICATION

(11) **EP 1 603 566 B1**
(45) Date of publication and mention of the grant of the patent: **21.01.2009**
(21) Application number: 04718960.0
(22) Date of filing: 10.03.2004
(51) Int. Cl.: A61K 31/4725, A61P 35/00

(54) **USE OF ISOQUINOLINE DERIVATIVES FOR TREATING CANCER AND MAP KINASE RELATED DISEASES**
VERWENDUNG VON ISOCHINOLIN-DERIVATEN ZUR BEHANDLUNG VON KREBS UND ERKRANKUNGEN IM ZUSAMMENHANG MIT MAP KINASE
UTILISATION DE DERIVES D'ISOQUINOLINE AUX FINS DU TRAITEMENT DE CANCERS ET D'ETATS PATHOLOGIQUES LIES A LA KINASE ASSOCIEE AUX MEMBRANES (MAP)

(30) Priority: 11.03.2003 US 453624 P
(43) Date of publication of application: 14.12.2005
(73) Proprietor: NOVARTIS AG, 4056 Basel (CH)
(72) Inventor: BATT, David, Bryant, Bridgewater, NJ 08807 (US); BOLD, Guido, CH-5073 Gipf-Oberfrick (CH); KIM, Sunkyu, Cambridge, MA 02139 (US); RAMSEY, Timothy, Michael, Sparta, NJ 07871 (US); SABIO, Michael, Lloyd, Ridgewood, NJ 07450 (US)
(74) Representative: de Weerd, Petrus G.W.
(86) International application number: PCT/EP2004/002460
(87) International publication number: WO 2004/080464

(56) References cited:
- WO-A-00/09495
- WO-A-01/23375
- WO-A-01/58899
- WO-A-03/059354
- US-A1- 2003 078 271
- US-A1- 2004 043 388
- US-A1- 2004 092 570

## Description

### Summary

The present invention relates to the discovery that certain compounds inhibit RAF kinase, a serine/threonine kinase that functions in the MAP kinase signaling pathway, and to the use of the compounds for the treatment of the disease characterized by excessive signaling in the MAP kinase signaling pathway, melanoma

### Background

Cells communicate various aspects of their extracellular environment to the nucleus by using various signal transduction pathways. Many of these signals are transmitted by protein kinases which activate various factors through the transfer of phosphate groups. Disruption of signal transduction by inhibiting appropriate kinase activity can have a clinical benefit as has been demonstrated by imatinib, an inhibitor of bcr-abl kinase, which is marketed as its mesylate salt under the brand GLEEVEC (in the United States) or GLIVEC.

The MAP kinase signaling pathway is known in the art as one of the pathways for growth factors to send their signal to proliferate from the extracellular environment to the cell nucleus. The growth factors activate transmembrane receptors located on the cell surface which in turn start a cascade whereby RAS is activated and recruits RAF kinase to the membrane where it is activated and in turn activates MEK kinase which then activates ERK kinase. Activated ERK kinase can move to the nucleus where it activates various gene transcription factors. Aberrations in this pathway can lead to altered gene transcription, cellular growth and contribute to tumorogenicity by negatively regulating apoptosis and transmitting proliferative and angiogenic signals. Inhibitors of RAF kinase have been shown to block signaling through the MAP kinase signaling pathway.

The RAF kinase family is known to have three members designated C-RAF, also known as RAF-1, B-RAF and A-RAF. It has been reported that B-RAF kinase is commonly activated by one of several somatic point mutations in human cancer, including 59% of the melanoma cell lines tested. See, Davies, H. et al, Nature 417, 949-954 (2002). Surprisingly, it has now been found that the compounds utilized in the method described herein are efficient inhibitors of RAF kinase, particularly C-RAF kinase and wild and mutated B-RAF kinases, particularly the V599E mutant B-RAF kinase.

The compounds utilized in the present method of treatment are described, for example, in U.S. Published Application 2002-0010191 and WO 01/58899, as antiangiogenic agents due to their ability to inhibit vascular endothelial growth factor. However, these publications do not suggest that the isoquinoline compounds possess RAF kinase inhibiting properties and do not suggest that the compounds would have the therapeutic benefits associated with RAF kinase inhibiting properties.

In WO 01/23375, substituted pyridines and pyridazines are disclosed which purport to have anti-angiogenesis activity and are therefore of use in VEGF - mediated conditions in both humans and other mammals. No mention is made of melanoma.

The RAF kinase inhibiting property of the compounds makes them useful as therapeutic agents for the treatment for proliferative diseases characterized by an aberrant MAP kinase signaling pathway, particularly many cancers characterized by overexpression of RAF kinase or an activating mutation of RAF kinase, such as melanoma having mutated B-RAF, especially wherein the mutated B-RAF is the V599E mutant. The present invention also provides a method of treating other conditions characterized by an aberrant MAP kinase signaling pathway, particularly where B-RAF is mutated, for example benign Nevi moles having mutated B-RAF, with the compounds.

### Description

The invention also relates to the use of a compound of formula I for the preparation of a medicament for the treatment of a melanoma disease characterized by excessive signaling through the MAP kinase signaling pathway.

The patient is a mammal, generally a human, suffering from a melanoma disease that is characterized by excessive signaling through the MAP kinase pathway. This can be measured by activation state specific antibodies to pathway members by methods such as Western blot analysis or immunohistochemistry. Such methods are known to those of skill in the art.

In general, the melanoma disease of the invention is characterized by excessive signaling through the MAP kinase signaling pathway is a proliferative disease, particularly a cancer characterized by increased RAF kinase activity, for example one which overexpresses wild-type B- or C-RAF kinase, or that expresses an activating mutant RAF kinase, for example a mutant B-RAF kinase. Mutated B-RAF kinase is especially prevalent in many melanomas.

In accordance with the present disclosure, a sample of diseased tissue is taken from the patient, for example, as a result of a biopsy or resection, and tested to determine whether the tissue produces a mutant RAF kinase, such as a mutant B-RAF kinase or overexpresses a wild-type RAF kinase, such as wild-type B- or C-RAF kinase. If the test indicates that mutant RAF kinase is produced or that a RAF kinase is overproduced in the diseased tissue, the patient is treated by administration of an effective RAF-inhibiting amount of a RAF inhibitor compound of claim 1.

Further in accordance with the present disclosure is the use of a compound of formula I described herein for the preparation of a medicament for the treatment of melanoma which comprises
(a) testing melanoma tissue from the patient to determine whether the melanoma tissue expresses mutant RAF kinase or overexpresses a wild-type RAF kinase and (b) treating the patient if the melanoma tissue is found to overexpress a wild type RAF kinase or express an activating mutant B-RAF kinase with an effective RAF kinase inhibiting amount of a compound of formula I.

However, it is also possible to downregulate the MAP kinase signaling pathway with a RAF kinase inhibiting compound if another kinase in the cascade is the cause of excessive signaling in the pathway. Thus, the present invention further relates to the use of a compound of claim 1 the manufacture of a medicament for the treatment of a disease characterized by excessive signaling in the MAP kinase signaling pathway attributed to a cause other than an activating mutation in or overexpression of a RAF kinase.

Tissue samples are tested by methods generally known in the art. For example, B-RAF mutations are detected by allele specific PCR, DHPLC, mass spectropscopy and overexpression of wild-type B- or C-RAF detected by immunohistochemistry, immunofluoresense, or Western blot analysis. A particularly useful method of detecting B-RAF mutations is the polymerase chain reaction based method described in Example D1. Similar methods are used to determine whether other kinases in the cascade are mutant or overexpressed.

A particularly important aspect of the present disclosure relates to a method of treating melanoma, which comprises (a) testing melanoma tissue from a patient to determine whether the melanoma tissue expresses mutant RAF kinase or overexpresses a wild-type RAF kinase and (b) treating the patient with an effective RAF kinase inhibiting amount of a RAFinhibiting compound described herein if the melanoma tissue is found to overexpress a wild type RAF kinase or express an activating mutant B-RAF kinase.

An important aspect of this disclosure relates to a method of treating melanoma, which comprises (a) testing melanoma tissue from a patient to determine whether the melanoma tissue overexpresses B-RAF kinase or C-RAF kinase activity and (b) treating the patient with an effective RAF kinase inhibiting amount of a RAF inhibiting compound described herein if the melanoma tissue is found to overexpress the B-RAF kinase or C-RAF kinase activity.

Another important aspect of this disclosure relates to a method of treating melanoma, which comprises (a) testing melanoma tissue from a patient to determine whether the melanoma tissue expresses mutant B-RAF kinase and (b) treating the patient with an effective RAF kinase inhibiting amount of a RAF inhibiting compound described herein if the melanoma tissue is found to express mutant B-RAF kinase.

Generally, the B-RAF kinase mutation is one of those described in the Davies *et al* article cited. These mutations are summarized in Table 1.

**Table 1**

| **B-RAF** | **protein change** |
|---|---|
| **mutation** | |
| G1388A | G463E |
| G1388T | G463V |
| G1394C | G465A |
| G1394A | G465E |
| G1394T | G465V |
| G1403C | G468A |
| G1403A | G468E |
| G1753A | E585K |
| T1782G | F594L |
| G1783C | G595R |
| C1786G | L596V |
| T1787G | L596R |
| T1796A | V599E |
| TG1796-97AT | V599D |

Thus, the present invention relates to the use described in claim 1 in treating a disease characterized by an activated mutant B-RAF kinase, which comprises detecting a mutation in the B-RAF kinase gene or protein in a tissue sample from a patient and treating the patient with an effective B-RAF kinase inhibiting compound, according to the claims.

A important aspect of this invention includes those instances wherein the mutant B-RAF kinase exhibits a mutation described in Table 1, especially the V599E mutation.

A particularly important aspect of this invention includes those instances wherein disease is melanoma and the mutant B-RAF kinase exhibits a mutation described in Table 1, especially the V599E mutation.

Accordingly, this invention includes the use of claim 1 for treating melanoma characterized by mutant B-RAF kinase, which comprises detecting a mutation in the B-RAF kinase gene selected from G1388A, G1388T, G1394C, G1394A, G1394T, G1403C, G1403A, G1753A, T1782G, G1783C, C1786G, T1787G, T1796A and TG1796-97AT, or corresponding mutation in the RAF kinase protein, in a tissue sample from a patient and treating the patient with an effective B-RAF kinase inhibiting compound described herein.

Within the context of the present disclosure, the general terms used herein to describe compounds of formula (I) have the following meanings, unless indicated otherwise.

The term "C₁ to ₇ denotes a radical having up to and including a maximum of 7, it is preferred up to and including a maximum of 4, carbon atoms, the radicals in question being unbranched or branched one or more times

Any reference to compounds, or salts in the plural is always to be understood as including one compound, or one salt.

Asymmetric carbon atoms which may be present (for example in compounds of formula I (or an N-oxide thereof) wherein n = 1 and R is lower alkyl) may have the (R), (S) or (R,S) configuration, preferably the (R) or (S) configuration. Substituents at a double bond or a ring may be in the cis (= Z) or trans (= E) form. Accordingly, the present compounds may be in the form of isomeric mixtures or in the form of pure isomers, preferably in the form of an enantiomerically pure diastereoisomer.

The index r is preferably 0 or 1.

The index n is preferably 0 or 1, especially 0. It may also be 2.

The index m is preferably 0, 1 or 2, especially 0, or also 1.

Of the ring members A, B, D, E and T in formula I, not more than three are to be N, and the others are CH or CQ. Preferably, the ring members A, B, D and E are each CH or CQ and T is N.

When G is a divalent group -CH₂-O-, -CH₂-S- or -CH₂-NH-, the methylene group is in each case bonded to the ring having the ring members A, B, D, E and T, while the hetero atom (O, S or NH) is bonded to the isoquinoline ring in formula I.

C₁ to 7 alkylene G may be branched or, preferably, unbranched and is especially branched or, preferably, unbranched C₁-C₄alkylene, especially methylene (-CH₂-), ethylene (-CH₂-CH₂-), trimethylene (-CH₂-CH₂-CH₂-) or tetramethylene (-CH₂-CH₂-CH₂-CH₂-). G is preferably methylene. C₁ to 7 alkylene G is preferably unsubstituted, but may be substituted by acyloxy, oxo, halogen or hydroxy.

Acyl in acyloxy-substituted C₁ to 7 alkylene G substituents is preferably arylcarbonyloxy, > wherein aryl is as defined below, especially benzoyloxy or alkanoyloxy, more especially benzoyloxy; acyloxy-substituted alkylene is especially benzoyloxy-substituted methylene.

G as hydroxy-substituted C₁ to 7 alkylene is preferably hydroxymethylene (-CH(OH)-).

A preferred oxo-substituted C₁ to 7 alkylene G substituents is carbonyl (-C(O)-).

Halogen-substituted C₁ to 7 alkylene G substituents are monohalo to perhalo substituted alkylene, such as difluoromethylene.

C₁ to 7 alkyl is especially C₁-C₄alkyl, for example n-butyl, sec-butyl, tert-butyl, n-propyl, iso-propyl or, especially, methyl or also ethyl, or, in the case of Y as C₁ to 7 alkyl, it may be especially isopentyl.

Aryl is preferably an aromatic radical having from 6 to 14 carbon atoms, such as phenyl, biphenyl, naphthyl, fluorenyl or phenanthrenyl, especially phenyl, the aryl radical being unsubstituted or substituted by one or more substituents, preferably up to three, especially one or two substituents, especially selected from amino, mono- or di-substituted amino, halogen, alkyl, substituted alkyl, hydroxy, etherified or esterified hydroxy, nitro, cyano, carboxy, esterified carboxy, alkanoyl, carbamoyl, N-mono- or N,N-di-substituted carbamoyl, amidino, guanidino, mercapto, sulfo, phenylthio, phenyl-C₁ to 7 alkylthio, alkylphenylthio, phenyl sulfinyl, phenyl C₁ to 7 alkylsulfinyl, alkylphenylsulfinyl, phenylsulfonyl, phenyl C₁ to 7 alkanesulfonyl, alkylphenylsulfonyl, C₁ to 7 alkenyl, such as ethenyl, phenyl, arylalkyl, such as benzyl or 1-methyl-1-phenyl-ethyl, C₁ to 7 alkylthio, such as methylthio C₁ to 7 alkyl silyl, such as trimethylsilyl C₁ to 7 alkanoyl, such as acetyl, unsubstituted or substituted cycloalkyl, C₁ to 7 alkylmercapto, such as methylmercapto (-S-CH₃), halo-C₁ to 7 alkylmercapto, such as trifluoromethylmercapto (-S-CF₃), C₁ to 7 alkanesulfonyl, halo-C₁ to 7 alkanesulfonyl, such as, especially, trifluoromethanesulfonyl, dihydroxybora (-B(OH)₂), heterocyclyl, and C₁ to 7 alkylenedioxy, such as methylenedioxy, bonded to adjacent carbon atoms of the ring or wherein two adjacent positions are substituted by alkylene or alkenylene to form a 5 to 7 membered ring that is fused to the aryl ring; aryl is preferably phenyl that is unsubstituted or substituted by one or two identical or different substituents from the group above especially halogen, especially fluorine or chlorine; C₁ to 7 alkyl, especially methyl, ethyl, propyl or t-butyl; halo-C₁ to 7 alkyl, especially trifluoromethyl; hydroxy; C₁ to 7 alkoxy, especially methoxy or ethoxy; phenyl-lower alkoxy, C₁ to 7 alkanoyl, such as acetyl, phenyloxy, halo-C₁ to 7 alkyloxy, such as trifluoromethoxy or 1,1,2,2-tetrafluoroethyloxy, C₁ to 7 alkoxycarbonyl, such as ethoxycarbonyl, C₁ to 7 alkylmercapto, such as methylmercapto, halo-C₁ to 7 alkylmercapto, such as trifluoromethylmercapto, hydroxy-C₁ to 7 alkyl, such as hydroxymethyl, C₁ to 7 alkanesulfonyl, such as methanesulfonyl, halo-C₁ to 7 alkanesulfonyl, such as trifluoromethanesulfonyl, phenylsulfonyl; more especially by one or two identical or different substituents selected from unsubstituted or substituted C₁ to 7 alkyl, especially methyl, t-butyl or trifluoromethyl and halogen, especially fluorine or chlorine.

Heteroaryl is preferably an unsaturated heterocyclic radical in the bonding ring and is preferably mono- or also bi- or tri-cyclic; wherein at least in the ring bonding to the radical of the molecule of formula I one or more, preferably from one to four, especially one or two, carbon atoms of a corresponding aryl radical have been replaced by a hetero atom selected from the group consisting of nitrogen, oxygen and sulfur, the bonding ring having preferably from 4 to 12, especially from 5 to 7, ring atoms; wherein heteroaryl is unsubstituted or substituted by one or more, especially from one to three, identical or different substituents from the group consisting of the substituents mentioned above as substituents of aryl; and is especially a heteroaryl radical selected from the group consisting of imidazolyl, thienyl, furyl, pyranyl, thianthrenyl, isobenzofuranyl, benzofuranyl, chromenyl, 2H-pyrrolyl, pyrrolyl, lower alkyl-substituted imidazolyl, benzimidazolyl, pyrazolyl, thiazolyl, isothiazolyl, oxazolyl, isoxazolyl, pyridyl, pyrazinyl, pyrimidinyl, pyridazinyl, indolizinyl, isoindolyl, 3H-indolyl, indolyl, indazolyl, triazolyl, tetrazolyl, purinyl, 4H-quinolizinyl, isoquinolyl, quinolyl, phthalazinyl, naphthyridinyl, quinoxalyl, quinazolinyl, cinnolinyl, pteridinyl, carbazolyl, phenanthridinyl, acridinyl, perimidinyl, phenanthrolinyl and furazanyl, each of those radicals being bonded *via* a ring having at least one hetero atom to the radical of the molecule of formula I; pyridyl is especially preferred.

The term "lower" is to be read as meaning "C₁₋₇" in the whole application.

Mono- or di-substituted amino is especially amino that is substituted by one or two identical or different radicals from lower alkyl, such as methyl; hydroxy-lower alkyl, such as 2-hydroxyethyl; phenyl-lower alkyl; lower alkanoyl, such as acetyl,; benzoyl; substituted benzoyl, wherein the phenyl radical is unsubstituted or, especially, is substituted by one or more, preferably one or two, substituents selected from nitro and amino, or also from halogen, amino, N-lower alkylamino, N,N-di-lower alkylamino, hydroxy, cyano, carboxy, lower alkoxycarbonyl, lower alkanoyl and carbamoyl; and phenyl-lower alkoxycarbonyl wherein the phenyl radical is unsubstituted or, especially, is substituted by one or more, preferably one or two, substituents selected from nitro and amino, or also from halogen, amino, N-lower alkylamino, N,N-di-lower alkylamino, hydroxy, cyano, carboxy, lower alkoxycarbonyl, lower alkanoyl and carbamoyl; and is preferably N-lower alkylamino, such as N-methylamino, hydroxy-lower alkylamino, such as 2-hydroxyethylamino, phenyl-lower alkylamino, such as benzylamino, N,N-di-lower alkylamino, N-phenyl-lower alkyl-N-lower alkylamino, N,N-di-lower alkylphenylamino, lower alkanoylamino, such as acetylamino, or a substituent selected from the group consisting of benzoylamino and phenyl-lower alkoxycarbonylamino, wherein in each case the phenyl radical is unsubstituted or, especially, is substituted by nitro or amino, or also by halogen, amino, N-lower alkylamino, N,N-di-lower alkylamino, hydroxy, cyano, carboxy, lower alkoxycarbonyl, lower alkanoyl or by carbamoyl, or alternatively or additionally to the preceding group of radicals, by aminocarbonylamino.

Halogen is especially fluorine, chlorine, bromine or iodine, more especially fluorine, chlorine or bromine, in particular fluorine and chlorine.

Alkyl has preferably up to a maximum of 12 carbon atoms and is especially lower alkyl, more especially methyl, or also ethyl, n-propyl, isopropyl or tert-butyl.

Substituted alkyl is especially lower alkyl, preferably methyl, which may contain one or more, especially up to three, substituents selected especially from the group consisting of halogen, especially fluorine, for example trifluromethyl or perfluoroalkyl generall, and also amino, N-lower alkylamino, N,N-di-lower alkylamino, N-lower alkanoylamino, hydroxy, cyano, carboxy, lower alkoxycarbonyl and phenyl-lower alkoxycarbonyl. Trifluoromethyl is an important substituted alkyl.

Etherified hydroxy is especially C₈-C₂₀alkyloxy, such as n-decyloxy, lower alkoxy (preferred), such as methoxy, ethoxy, isopropyloxy or n-pentyloxy, phenyl-lower alkoxy, such as benzyloxy, or also phenyloxy, or, alternatively or additionally to the preceding group, C₈-C₂₀alkyloxy, such as n-decyloxy, halo-lower alkoxy, such as trifluoromethyloxy or 1,1,2,2-tetrafluoroethoxy.

Esterified hydroxy is especially lower alkanoyloxy, benzoyloxy, lower alkoxycarbonyloxy, such as tert-butoxycarbonyloxy, or phenyl-lower alkoxycarbonyloxy, such as benzyloxycarbonyloxy.

Esterified carboxy is especially lower alkoxycarbonyl, such as tert-butoxycarbonyl or ethoxycarbonyl, phenyl-lower alkoxycarbonyl or phenyloxycarbonyl.

Alkanoyl is especially alkyl-carbonyl, more especially lower alkanoyl, for example acetyl.

N-Mono- or N,N-di-substituted carbamoyl is especially substituted at the terminal nitrogen by one or two substituents lower alkyl, phenyl-lower alkyl or hydroxy-lower alkyl.

Alkylphenylthio is especially lower alkylphenylthio.

Alkylphenylsulfinyl is especially lower alkylphenylsulfinyl.

Alkylphenylsulfonyl is especially lower alkylphenylsulfonyl.

Pyridyl Y is preferably 3- or 4-pyridyl.

Unsubstituted or substituted cycloalkyl is preferably C₃-C₈cycloalkyl which is unsubstituted or is substituted in the same manner as aryl, especially as defined for phenyl. Preference is given to cyclohexyl, or also cyclopentyl or cyclopropyl. Preference is given also to 4-lower alkyl-cyclohexyl, such as 4-tert-butylcyclohexyl.

If present, Z is preferably amino, hydroxy-lower alkylamino, such as 2-hydroxyethylamino, lower alkanoylamino, such as acetylamino, nitrobenzoylamino, such as 3-nitrobenzoylamino, aminobenzoylamino, such as 4-aminobenzoylamino, phenyl-lower alkoxycarbonylamino, such as benzyloxycarbonylamino, or halogen, such as bromine; preferably only one substituent is present (m = 1), especially one of the last-mentioned substituents, especially halogen. Very special preference is given to a compound of formula I (or an N-oxide thereof) wherein Z is not present (m = 0).

Heterocyclyl is especially a five- or six-membered heterocycle having 1 or 2 hetero atoms selected from the group consisting of nitrogen, oxygen and sulfur, which heterocycle may be unsaturated or fully or partially saturated, and is unsubstituted or substituted, especially by lower alkyl, such as methyl; preference is given to a radical selected from 2-methyl-pyrimidin-4-yl, oxazol-5-yl, 2-methyl-1,3-dioxolan-2-yl, 1H-pyrazol-3-yl and 1-methyl-pyrazol-3-yl.

Aryl in the form of phenyl that is substituted by lower alkylenedioxy, such as methylenedioxy, bonded to two adjacent carbon atoms is preferably 3,4-methylenedioxyphenyl.

An N-oxide of a compound of formula I is preferably an N-oxide in which an isoquinoline ring nitrogen or a nitrogen in the ring having the ring members A, B, D and E carries an oxygen atom, or more than one of the mentioned nitrogen atoms carry an oxygen atom.

Salts are especially the pharmaceutically acceptable salts of compounds of formula I (or an N-oxide thereof).

Such salts are formed, for example, by compounds of formula I (or an N-oxide thereof) having a basic nitrogen atom as acid addition salts, preferably with organic or inorganic acids, especially the pharmaceutically acceptable salts. Suitable inorganic acids are, for example, hydrohalic acids, such as hydrochloric acid; sulfuric acid; or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, 2-hydroxybutyric acid, gluconic acid, glucosemonocarboxylic acid, fumaric acid, succinic acid, adipic acid, pimelic acid, suberic acid, azelaic acid, malic acid, tartaric acid, citric acid, glucaric acid, galactaric acid, amino acids, such as glutamic acid, aspartic acid, N-methylglycine, acetylaminoacetic acid, N-acetylasparagine, N-acetylcysteine, pyruvic acid, acetoacetic acid, phosphoserine, 2- or 3-glycerophosphoric acid, maleic acid, hydroxymaleic acid, methylmaleic acid, cyclohexanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, glucuronic acid, galacturonic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalenedisulfonic acid, N-cyclohexylsulfamic acid, N-methyl-, N-ethyl- or N-propyl-sulfamic acid, or other organic protonic acids, such as ascorbic acid.

When negatively charged radicals, such as carboxy or sulfo, are present, salts with bases can also be formed, for example metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethylpiperidine or N,N'-dimethyl-piperazine.

When a basic group and an acid group are present in the same molecule, a compound of formula I (or an N-oxide thereof) can also form internal salts.

For isolation or purification it is also possible to use pharmaceutically unacceptable salts, for example picrates or perchlorates. Only the pharmaceutically acceptable salts or the free compounds (optionally in the form of pharmaceutical compositions) are used therapeutically, and those are therefore preferred.

In view of the close relationship between the compounds in free form and in the form of their salts, including also those salts which can be used as intermediates, for example in the purification of the novel compounds or for their identification, hereinbefore and hereinafter any reference to the free compounds is also to be understood as including the corresponding salts, as appropriate and expedient.

The present invention further relates to the use according to the claims by inhibiting RAF kinase, which comprises contacting the RAF kinase with a compound of formula (I). Preferably, the RAF kinase is B-or C-RAF kinase, or a mutant RAF kinase, especially a mutant B-RAF kinase, particularly the V599E mutant.

The compounds of formula I (or N-oxides thereof) have valuable pharmacological properties, as described above.

A compound of formula I (or an N-oxide thereof) can be administered on its own or in combination with one or more other therapeutic agents, it being possible for fixed combinations to be used or for a compound according to the invention and one or more other therapeutic agents to be administered in a staggered manner over time or independently of one another, or the combined administration of fixed combinations and of one or more other therapeutic agents is possible. In particular, the administration of a compound of formula I (or an N-oxide thereof) for tumour treatment can be carried out, alongside or additionally, in combination with chemotherapy (combination with one or more other chemotherapeutic agents, especially cytostatics, or with hormones or compounds having a hormone-like activity), radiotherapy, immunotherapy, surgical treatment or combinations thereof. Long-term therapy is also possible, as is adjuvant therapy in conjunction with other treatment methods, such as those just mentioned. Treatment to maintain the status of a patient after tumour remission or even chemopreventive treatment, for example in the case of at-risk patients, is also possible.

There come into consideration as therapeutic agents with which the compounds according to the invention can be combined especially one or more antiproliferative, cytostatic or cytotoxic compounds, for example one or more chemotherapeutic agents selected from the group comprising an inhibitor of polyamine biosynthesis, an inhibitor of a different protein kinase, especially protein kinase C, or of a tyrosine protein kinase, such as epidermal growth factor receptor protein tyrosine kinase, an inhibitor of a growth factor, such as vascular endothelial growth factor, a cytokine, a negative growth regulator, such as TGF-β or IFN-β, an aromatase inhibitor, hormones or hormone analogues, and a conventional cytostatic agent.

Compounds according to the invention are intended not only for the (prophylactic and, preferably, therapeutic) treatment of human beings, but also for the treatment of other warm-blooded animals, for example of commercially useful animals, for example rodents, such as mice, rabbits or rats, or guinea pigs.

In general, the invention relates also to the use of a compound of formula I (or an N-oxide thereof) in inhibiting RAF kinase activity according to the claims.

A compound of formula I (or an N-oxide thereof) can also be used for diagnostic purposes, for example in order that tumours obtained from warm-blooded animals, especially human beings, as the original "host" and transplanted into mice, can be examined for reduced growth after addition of such a compound, in order thus to study their sensitivity to the compound in question, thus allowing possible methods of treatment for a tumour disease in the original host to be ascertained and determined better: this is not part of the invention

In the groups of preferred compounds of formula I mentioned below, definitions of substituents from the above-mentioned general definitions may expediently be used, for example in order to replace more general definitions by definitions that are more specific or, especially, by definitions that are indicated as being preferred; preference is in each case given to the definitions indicated above as being preferred or mentioned by way of example.

Preference is given to a compound of formula I wherein
r is from 0 to 2, preferably 0;
n is 0 or 1;
m is 1 or, especially, 0;
A, B, D and E are each CH and T is N, or A, D and E are each CH and B and T are N, or A, B, E and T are CH and D is N, or A, T, D and E are CH and B is N; particularly wherein A, B, D and E are each CH and T is N, or A, D and E are each CH and B and T are N.
G is lower alkylene, especially methylene or ethylene (-CH₂-CH₂-), -CH₂-NH-, -CH₂-O-, hydroxymethylene or benzoyloxy-methylene;
Q is methyl which is bonded to A, to D or to A and D;
R is H or lower alkyl, especially H or methyl;
X is -NR-, oxa or thia, expecially -NH-
Y is phenyl that is unsubstituted or substituted by one or two identical or different substituents selected from the group consisting of amino; lower alkanoylamino, especially acetylamino; halogen, especially fluorine, chlorine or bromine; unsubstituted or substituted lower alkyl, especially methyl, ethyl, propyl, t-butyl or halo-lower alkyl, especially trifluoromethyl; hydroxy; lower alkoxy, especially methoxy or ethoxy; phenyl-lower alkoxy, especially benzyloxy; cyano, lower alkenyl, such as ethenyl, C₈-C₁₂alkoxy, especially n-decyloxy, lower alkoxycarbonyl, such as tert-butoxycarbonyl, carbamoyl, lower alkylcarbamoyl, such as N-methyl- or N-tert-butyl-carbamoyl, lower alkanoyl, such as acetyl, phenyloxy, halo-lower alkyloxy, such as trifluoromethoxy or 1,1,2,2-tetrafluoroethyloxy, lower alkoxycarbonyl, such as ethoxycarbonyl, lower alkylmercapto, such as methylmercapto, halo-lower alkylmercapto, such as trifluoromethylmercapto, hydroxy-lower alkyl, such as hydroxymethyl or 1-hydroxymethyl, lower alkanesulfonyl, such as methanesulfonyl, halo-lower alkanesulfonyl, such as trifluoromethanesulfonyl, phenylsulfonyl and lower alkylenedioxy, such as methylenedioxy, bonded to two adjacent carbon atoms or wherein two adjacent positions are substituted by alkylene or alkenylene to form a 5 to 7 membered ring that is fused to the phenyl ring, especially by one or two substituents selected from halogen, such as chlorine or bromine, unsubstituted lower alkyl, such as methyl, and halo-substitued lower alkyl, such as trifluoromethyl, Y is especially phenyl or phenyl that is substituted by one or two identical or different substituents, which are especially halogen, especially fluoro or chloro, and/or unsubstitued or substituted lower alkyl;
Z is amino; N-lower alkylamino, such as N-methylamino; hydroxy-lower alkylamino, such as 2-hydroxyethylamino; phenyl-lower alkylamino, such as benzylamino; N,N-di-lower alkylamino; N-phenyl-lower alkyl-N-lower alkylamino; N,N-di-lower alkylphenylamino; lower alkanoylamino, such as acetylamino; or a substituent selected from the group consisting of benzoylamino and phenyl-lower alkoxycarbonylamino, wherein the phenyl radical in each case is unsubstituted or, especially, is substituted by nitro or by amino, or also by halogen, amino, N-lower alkylamino, N,N-di-lower alkylamino, hydroxy, cyano, carboxy, lower alkoxycarbonyl, lower alkanoyl or by carbamoyl; or Z is halogen, especially bromine; more especially amino, acetylamino, nitrobenzoylamino, aminobenzoylamino, 2-hydroxyethylamino, benzyloxycarbonylamino or bromine; and or a salt or N-oxide thereof.

Special preference is given to a compound of formula I wherein
r is 0;
n is 0;
m is 0;
B, D, E and T are CH and A is N (3-pyridyl), or especially wherein A, B, D and E are each CH and T is N (4-pyridyl);
G is lower alkylene, especially methylene;
X is -NR- especially -NH-;
Y is phenyl that is unsubstituted or substituted by one or two identical or different substituents selected from the group consisting of halogen, especially fluorine or, more especially, chlorine or bromine; lower alkyl, especially methyl; and halo-lower alkyl, especially trifluoromethyl; especially 4-chlorophenyl, 2-, 3- or 4-methylphenyl, 4-chloro-5-trifluoromethylphenyl, 3-bromo-5-trifluoromethylphenyl, or more especially 3,5-dimethylphenyl; or also 4-methyl-3-iodophenyl, 3,4-bis(trifluoromethyl)phenyl or 3-bromo-4-ethylphenyl;
or a salt thereof.

Special preference is given also to a compound of formula I wherein
r is 0;
n is from 0 to 2;
m is 0;
A, B, D and E are each CH and T is N;
G is methylene;
R is H;
X is -NR-, especially -NH-; and
Y is phenyl that is unsubstituted or substituted by halogen, especially fluorine or chlorine, or by lower alkyl, such as methyl or trifluromethyl, lower alkoxy, especially methoxy, such as 4-chlorophenyl, 4-methoxyphenyl or 4-trifluoromethoxyphenyl; naphthyl; cyclohexyl that is unsubstituted or substituted by lower alkyl, especially by tert-butyl, such as 4-tert-butylcyclohexyl; indolyl that is unsubstituted or substituted by halogen, especially by fluorine, especially 6-fluoroindol-3-yl; or lower alkyl, especially isopentyl;
or a salt thereof where a salt-forming group is present.

In particular, preference is given also to a compound of formula I wherein
r is 0;
n is 0;
m is 0;
B, D, E and T are CH and A is N or A, B, D and E are each CH and T is N;
G is lower alkylene;
X is -NH-;
Y is phenyl that is unsubstituted or substituted by one or two identical or different substituents selected from the group consisting of halogen and lower alkyl.

Preference is given also to a compound of formula I wherein
r is 0;
n is from 0 to 2;
m is 0;
A, B, D and E are each CH and T is N;
G is methylene;
R is H;
X is -NR; and
Y is phenyl that is unsubstituted or substituted by halogen, lower alkyl, lower alkoxy or cyclohexyl that is unsubstituted or substituted by lower alkyl.

In particular, especially useful compounds include those wherein
r is 0;
n is 0;
m is 0;
A, B, D and E are each CH and T is N;
G is methylene;
X -NH-; and
Y is phenyl that is substituted by one or two identical or different substituents selected from halogen and lower alkyl. Special preference is given to such compounds wherein Y is phenyl that is substituted in the 4-position by t-butyl or trifluoromethyl.

The compounds according to the invention can be prepared by processes known *per se* for other compounds, especially by
a) reacting a compound of formula II 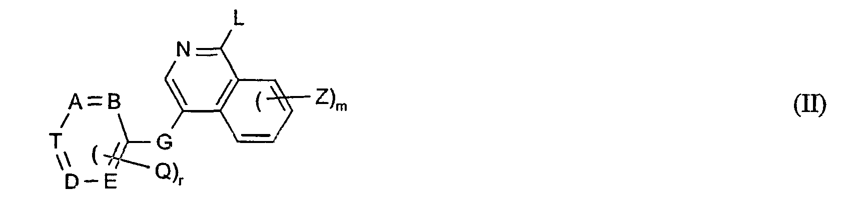 wherein r, m, A, B, D, E, T, G, Q and Z are as defined for a compound of formula I and L is a nucleofugal leaving group, with a compound of formula III 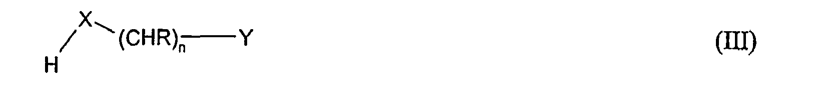 wherein n, R, X and Y are as defined for a compound of formula I, functional groups in the compounds of formula II and of formula III that are not to take part in the reaction being in protected form, if necessary, and removing any protecting groups that are present, wherein the starting compounds mentioned in process a) may also be in the form of salts where a salt-forming group is present and reaction in the salt form is possible;
   and, if desired, converting a resulting compound of formula I, or an N-oxide thereof, into a different compound of formula I or an N-oxide thereof, converting a free compound of formula I, or an N-oxide thereof, into a salt, converting a resulting salt of a compound of formula I, or of an N-oxide thereof, into the free compound or into a different salt, and/or separating a mixture of isomeric compounds of formula I, or its N-oxide, into the individual isomers.

### Detailed description of the process variants:

In the following, more detailed description of the preparation process, r, n, m, A, B, D, E, G, Q, R, X, Y and Z and are as defined for compounds of formula I, unless indicated otherwise.

### Process a)

In the compound of formula II, a nucleofugal leaving group L is especially halogen, more especially bromine, iodine or, very especially, chlorine.

The reaction between the compound of formula II and the compound of formula III takes place in suitable inert polar solvents, especially alcohols, for example lower alkanols, such as methanol, propanol or, especially, ethanol or n-butanol, or it takes place in a melt without the addition of a solvent, especially when one of the reactants is in liquid form. The reaction takes place at elevated temperatures, preferably from approximately 60°C to reflux temperature, for example under reflux conditions or at a temperature of from approximately 90 to approximately 110°C. The compound of formula III can also be used in the form of a salt, for example in the form of an acid addition salt with a strong acid, such as a hydrogen halide, for example in the form of the hydrochloride salt, or the corresponding acid, for example hydrochloric acid, can be added in a suitable solvent, for example an ether, such as dioxane.

Where one or more other functional groups, for example carboxy, hydroxy, amino or mercapto, in a compound of formula II and/or III are present in protected form or must be present in protected form because they are not to take part in the reaction, the protecting groups are groups which are customarily used in the synthesis of peptide compounds, but also in the synthesis of cephalosporins and penicillins as well as of nucleic acid derivatives and sugars. The protecting groups may already be present in the precursors and are to protect the functional groups in question against undesired secondary reactions, such as acylations, etherifications, esterifications, oxidations, solvolysis and the like. The protecting groups for functional groups in starting materials whose reaction is to be avoided, especially carboxy, amino, hydroxy and mercapto groups, include especially those protecting groups (conventional protecting groups) which are customarily used in the synthesis of peptide compounds, cephalosporins, penicillins or nucleic acid derivatives and sugars. The protecting groups may already be present in the precursors and are to protect the functional groups in question against undesired secondary reactions, such as acylations, etherifications, esterifications, oxidations, solvolysis, etc.. In some cases the protecting groups can cause the reactions to proceed selectively, for example stereoselectively. It is a characteristic of protecting groups that they can be removed easily, that is to say without undesired secondary reactions, for example by solvolysis, by reduction, by photolysis or enzymatically, for example also under conditions analogous to physiological conditions, and that they are not present in the end products. The person skilled in the art will know or can readily find out which protecting groups are suitable in the reactions mentioned hereinbefore and hereinafter.

The protection of functional groups by means of such protecting groups, the protecting groups themselves, and reactions for their removal are described, for example, in standard works such as J. F. W. McOmie, "Protective Groups in Organic Chemistry", Plenum Press, London and New York 1973, in Th. W. Greene, "Protective Groups in Organic Synthesis", Wiley, New York 1981, in "The Peptides"; Volume 3 (E. Gross and J. Meienhofer, eds.), Academic Press, London and New York 1981, in "Methoden der organischen Chemie", Houben Weyl, 4th edition, Volume 15/I, Georg Thieme Verlag, Stuttgart 1974, in H.-D. Jakubke and H. Jescheit, "Aminosäuren, Peptide, Proteine", Verlag Chemie, Weinheim, Deerfield Beach and Basle 1982, and in Jochen Lehmann, "Chemie der Kohlenhydrate: Monosaccharide und Derivate", Georg Thieme Verlag, Stuttgart 1974.

Protecting groups mentioned in the Examples are preferably introduced and, if required, removed analogously to the mentioned methods.

### Additional process steps

In the additional process steps, which are carried out if desired, functional groups in the starting compounds that are not to take part in the reaction may be present in unprotected form or in protected form, for example protected by one or more of the protecting groups mentioned above under process a). All or some of the protecting groups are then removed by one of the methods mentioned under process a).

Salts of compounds of formula I (or an N-oxide thereof) having a salt-forming group can be prepared in a manner known *per* se. For example, acid addition salts of compounds of formula I or their N-oxides can be obtained, for example, by treatment with an acid or a suitable anion exchange reagent. It is also possible to convert salts having two acid molecules (for example a dihalide of a compound of formula I (or of an N-oxide thereof)) into salts having one acid molecule per compound of formula I (or N-oxide thereof) (for example into a monohalide); that can be achieved, for example, by heating to the molten state or, for example, by heating in solid form under a high vacuum at elevated temperature, for example from 130 to 170°C, one molecule of the acid being expelled per molecule of a compound of formula I (or of an N-oxide thereof).

Salts can be converted into the free compounds in customary manner, for example by treatment with a suitable basic agent, for example with alkali metal carbonates, hydrogen carbonates or hydroxides, for example potassium carbonate or sodium hydroxide.

Stereoisomeric mixtures, for example mixtures of diastereoisomers, can be separated into the corresponding isomers in a manner known *per se* by means of suitable separating procedures. For example, diastereoisomeric mixtures can be separated into the individual diastereoisomers by fractional crystallisation, chromatography, solvent partitioning and the like. The separation may be carried out either at the stage of one of the starting materials or in the case of the compounds of formula I themselves. Enantiomers can be separated by formation of diastereoisomeric salts, for example by salt formation with an enantiomerically pure chiral acid, or by chromatographic methods, for example by chromatography, e.g. HPLC, on chromatographic carrier materials with chiral ligands.

A compound of formula I can be converted into a corresponding N-oxide. The reaction is carried out with a suitable oxidising agent, preferably a peroxide, for example m-chloroperbenzoic acid, in a suitable solvent, for example a halogenated hydrocarbon, such as chloroform or methylene chloride, or in a lower alkanecarboxylic acid, such as acetic acid, preferably at a temperature of from 0°C to the boiling temperature of the reaction mixture, especially approximately room temperature.

A compound of formula I (or an N-oxide thereof) wherein Z is lower alkanoylamino can be hydrolysed to the corresponding amino compound (Z = amino), for example by hydrolysis with an inorganic acid, especially hydrochloric acid (HCl), in aqueous solution, it being possible to add further solvents, preferably at elevated temperature, for example under reflux.

A compound of formula I (or an N-oxide thereof) wherein Z is amino substituted by one or two identical or different radicals selected from lower alkyl, hydroxy-lower alkyl and phenyl-lower alkyl can be converted into the compound that is correspondingly substituted at the amino group, for example, by reaction with a lower alkyl halide, a hydroxy-lower alkyl halide, which is hydroxy-protected if necessary (see process a)), or a phenyl-lower alkyl halide under reaction conditions analogous to those mentioned under process a). For the introduction of 2-hydroxy-lower alkyl substituents at the amino group Z, addition starting from an epoxide (for example ethylene oxide) is also possible. The addition is carried out especially in aqueous solution and/or in the presence of polar solvents, such as alcohols, for example methanol, ethanol, isopropanol or ethylene glycol, ethers, such as dioxane, amides, such as dimethyl formamide, or phenols, such as phenol, also under anhydrous conditions, in apolar solvents, such as benzene and toluene, or in benzene/water emulsions, optionally in the presence of acid or basic catalysts, for example of alkaline solutions, such as sodium hydroxide solution, or in the presence of hydrazine-doped solid phase catalysts, such as aluminium oxide, in ethers, for example diethyl ether, generally at temperatures of approximately from 0°C to the boiling temperature of the reaction mixture in question, preferably at from 20°C to reflux temperature, where appropriate under elevated pressure, for example in a bomb tube, whereby the boiling temperature may also be exceeded, and/or under an inert gas, such as nitrogen or argon. Reductive alkylation of an amino group Z with a lower alkanealdehyde, a phenyl-lower alkanealdehyde or a hydroxy-lower alkanealdehyde, which is hydroxy-protected if necessary, is also possible. The reductive alkylation preferably takes place with hydrogenation in the presence of a catalyst, especially a noble metal catalyst, such as platinum or, especially, palladium, which is preferably bonded to a support material, such as carbon, or a heavy metal catalyst, such as Raney nickel, at normal pressure or at pressures of from 0.1 to 10 megapascals (MPa), or with reduction by means of complex hydrides, such as boron hydrides, especially alkali metal cyanoborohydrides, for example sodium cyanoborohydride, in the presence of a suitable acid, preferably of a relatively weak acid, such as a lower alkanecarboxylic acid or, especially, a sulfonic acid, such as p-toluene-sulfonic acid; in customary solvents, for example alcohols, such as methanol or ethanol, or ethers, for example cyclic ethers, such as tetrahydrofuran, in the absence or presence of water.

In a compound of formula I (or an N-oxide thereof), an amino group Z can be converted by acylation into an amino group that is substituted by lower alkanoyl, benzoyl, substituted benzoyl or by phenyl-lower alkoxycarbonyl wherein the phenyl radical is unsubstituted or substituted. The corresponding acids contain a free carboxy group or are in the form of reactive acid derivatives thereof, for example in the form of the derived activated esters or reactive anhydrides, also reactive cyclic amides. The reactive acid derivatives can also be formed *in situ.* Activated esters are especially esters that are unsaturated at the linking carbon atom of the radical to be esterified, for example of the vinyl ester type, such as vinyl esters (obtainable, for example, by transesterification of a corresponding ester by vinyl acetate; activated vinyl ester method), carbamoyl esters (obtainable, for example, by treating the corresponding acid with an isoxazolium reagent; 1,2-oxazolium or Woodward method), or 1-lower alkoxyvinyl esters (obtainable, for example, by treating the corresponding acid with a lower alkoxyacetylene; ethoxyacetylene method), or esters of the amidino type, such as N,N'-disubstituted amidino esters (obtainable, for example, by treating the corresponding acid with a suitable N,N'-disubstituted carbodiimide, for example N,N'-dicyclohexylcarbodiimide or, especially, N-(3-dimethylaminopropyl)-N'-ethylcarbodiimide; carbodiimide method) or N,N-disubstituted amidino esters (obtainable, for example, by treating the corresponding acid with an N,N-disubstituted cyanamide; cyanamide method), suitable aryl esters, especially phenyl esters suitably substituted by electrophilic substituents (obtainable, for example, by treating the corresponding acid with a suitably substituted phenol, for example 4-nitrophenol, 4-methylsulfonylphenol, 2,4,5-trichlorophenol, 2,3,4,5,6-pentachlorophenol or 4-phenyldiazophenol, in the presence of a condensing agent, such as N,N'-dicyclohexylcarbodiimide; activated aryl esters method), cyanomethyl esters (obtainable, for example, by treating the corresponding acid with chloroacetonitrile in the presence of a base; cyanomethyl esters method), thioesters, especially unsubstituted or substituted, for example nitro-substituted, phenylthio esters (obtainable, for example, by treating the corresponding acid with unsubstituted or substituted, for example nitro-substituted, thiophenols, *inter alia* by means of the anhydride or carbodiimide method; activated thiolesters method), or, especially, amino or amido esters (obtainable, for example, by treating the corresponding acid with an N-hydroxyamino or N-hydroxyamido compound, for example N-hydroxysuccinimide, N-hydroxypiperidine, N-hydroxyphthalimide, N-hydroxy-5-norbornene-2,3-dicarboxylic acid imide, 1-hydroxybenztriazole or 3-hydroxy-3,4-dihydro-1,2,3-benztriazin-4-one, for example by the anhydride or carbodiimide method; activated N-hydroxy esters method). Internal esters, for example y-lactones, can also be used. Anhydrides of acids may be symmetrical or, preferably, mixed anhydrides of those acids, for example anhydrides with inorganic acids, such as acid halides, especially acid chlorides (obtainable, for example, by treating the corresponding acid with thionyl chloride, phosphorus pentachloride, phosgene or oxalyl chloride; acid chloride method), azides (obtainable, for example, from a corresponding acid ester *via* the corresponding hydrazide and treatment thereof with nitrous acid; azide method), anhydrides with carbonic acid semiesters, for example carbonic acid lower alkyl semiesters (especially chloroformic acid methyl esters) (obtainable, for example, by treating the corresponding acid with chloroformic acid lower alkyl esters or with a 1-lower alkoxycarbonyl-2-lower alkoxy-1,2-dihydroquinoline; mixed Q-alkylcarbonic acid anhydrides method), or anhydrides with dihalogenated, especially dichlorinated, phosphoric acid (obtainable, for example, by treating the corresponding acid with phosphorus oxychloride; phosphorus oxychloride method), anhydrides with other phosphoric acid derivatives (for example those which can be obtained with phenyl N-phenylphosphoramidochloridate, or by reacting alkylphosphoric acid amides in the presence of sulfonic acid anhydrides and/or racemisation-reducing additives, such as N-hydroxybenzotriazole, or in the presence of cyanophosphonic acid diethyl ester) or with phosphorous acid derivatives, or anhydrides with organic acids, such as mixed anhydrides with organic carboxylic acids (obtainable, for example, by treating the corresponding acid with an unsubstituted or substituted lower alkane- or phenyl-lower alkane-carboxylic acid halide, for example phenylacetic acid, pivalic acid or trifluoroacetic acid chloride; mixed carboxylic acid anhydrides method) or with organic sulfonic acids (obtainable, for example, by treating a salt, such as an alkali metal salt, of the corresponding acid with a suitable organic sulfonic acid halide, such as lower alkane- or aryl-, for example methane- or p-toluene-sulfonic acid chloride; mixed sulfonic acid anhydrides method) as well as symmetrical anhydrides (obtainable, for example, by condensing the corresponding acid in the presence of a carbodiimide or of 1-diethylaminopropyne; symmetrical anhydrides method). Suitable cyclic amides are especially amides with five-membered diazacycles of aromatic nature, such as amides with imidazoles, for example imidazole (obtainable, for example, by treating the corresponding acid with N,N'-carbonyldiimidazole; imidazole method), or pyrazole, for example 3,5-dimethylpyrazole (obtainable, for example, via the acid hydrazide by treatment with acetylacetone; pyrazolide method). As mentioned, derivatives of carboxylic acids which are used as acylating agents can also be formed *in situ.* For example, N,N'-disubstituted amidino esters can be formed *in situ* by reacting the mixture of the starting material of formula I and the acid used as acylating agent in the presence of a suitable N,N'-disubstituted carbodiimide, for example N,N'-dicyclohexylcarbodiimide or, especially, N-(3-dimethylaminopropyl)-N'-ethylcarbodiimide. Furthermore, amino or amido esters of the acids used as acylating agent can be formed in the presence of the starting material of formula I to be acylated, by reacting a mixture of the corresponding acid and amino starting materials in the presence of an N,N'-disubstituted carbodiimide, for example N,N'-dicyclohexylcarbodiimide, and of an N-hydroxyamine or N-hydroxyamide, for example N-hydroxysuccinimide, optionally in the presence of a suitable base, for example 4-dimethylaminopyridine. Moreover, activation can be achieved *in situ* by reaction with N,N,N',N'-tetraalkyluronium compounds, such as O-benztriazol-1-yl-N,N,N',N'-tetra-methyluronium hexafluorophosphate, O-(1,2-dihydro-2-oxo-1-pyridyl)-N,N,N',N'-tetramethyluronium tetrafluoroborate (in the absence or presence of 1,8-diazabicyclo[5.4.0]undec-7-ene-(1,5,5)) or O-(3,4-dihydro-4-oxo-1,2,3-benztriazolin-3-yl)-N,N,N',N'-tetramethyluronium tetrafluoroborate. Finally, phosphoric acid anhydrides of the carboxylic acids can be prepared *in situ* by reacting an alkylphosphoric acid amide, such as hexamethylphosphoric acid triamide, in the presence of a sulfonic acid anhydride, such as 4-toluenesulfonic acid anhydride, with a salt, such as a tetrafluoroborate, for example sodium tetrafluoroborate, or with a different derivative of hexamethylphosphoric acid triamide, such as benzotriazol-1-yl-oxy tris(dimethylamino)phosphonium hexafluoride, preferably in the presence of a racemisation-reducing additive, such as N-hydroxybenztriazole. If desired, an organic base is added, preferably a tertiary amine, for example a tri-lower alkylamine, especially ethyldiisopropylamine or, more especially, triethylamine, and/or a heterocyclic base, for example 4-dimethylaminopyridine or, preferably, N-methylmorpholine or pyridine. The condensation is preferably carried out in an inert, aprotic, preferably anhydrous solvent or solvent mixture, for example in a carboxylic acid amide, for example formamide or dimethylformamide, a halogenated hydrocarbon, for example methylene chloride, carbon tetrachloride or chlorobenzene, a ketone, for example acetone, a cyclic ether, for example tetrahydrofuran or dioxane, an ester, for example ethyl acetate, or a nitrile, for example acetonitrile, or in a mixture thereof, where appropriate at reduced or elevated temperature, for example in a temperature range of from approximately -40°C to approximately +100°C, preferably from approximately -10°C to approximately +70°C, where arylsulfonyl esters are used also at approximately from +100°C to +200°C, especially at temperatures of from 10 to 30°C, and, where appropriate, under an inert gas atmosphere, for example a nitrogen or argon atmosphere. Aqueous, for example alcoholic, solvents, e.g. ethanol, or aromatic solvents, e.g. benzene or toluene, are also possible.

A nitro group Z in a compound of formula I can be reduced to an amino group, for example by reduction with metals or selective hydrogenation; for example by reaction with magnesium/ammonium sulfate in a water/alcohol mixture, such as methanol/water, at elevated temperature, for example from 30 to 60°C (see Synth. Commun. 25(2), 4025-4028 (1995)); by reaction with zinc/borohydride in an acid amide, such as dimethylformamide, at temperatures below room temperature, for example at approximately 0°C; by reaction with 1,1'-dioctyl-4,4'-bipyridinium dibromide/sodium tetrathionate/potassium carbonate in water/halogenated hydrocarbon mixtures, for example water/methylene chloride mixtures, at elevated temperature, for example from 25 to 35°C (see Tetrahedron Lett. 34(46), 7445-7446 (1993)); with sodium borohydride on Amberlyte IRA-400 ion exchanger in the chloride form in an alcohol, such as methanol/water, at preferred temperatures of from 0 to 40°C (see Synthetic Commun. 19(5/6), 805-811 (1989)); with potassium borohydride in a halogenated hydrocarbon/alcohol mixture, for example methylene chloride/methanol, at preferred temperatures of from 10 to 35°C (see Synthetic Commun. 19(17), 3047-3050 (1989)); with sodium borohydride in dioxane; with borane in tetrahydrofuran; by hydrogenation in the presence of Pd/C in an alcohol at a preferred temperature of from 0 to 35°C and in the presence of ammonium formate (see Tetrahedron Lett. 25(32), 3415-3418 (1989)); with titanium tetrachloride/lithium aluminium hydride or titanium tetrachloride/magnesium in an ether, such as tetrahydrofuran (see Bull. Chem. Soc. Belg. 97(1), 51-53 (1988)); or with ferric ammonium chloride/water at elevated temperature, preferably under reflux (Synth. Commun. 22, 3189-3195 (1992)).

In a compound of formula I wherein G is acyloxy-substituted lower alkyl and the other radicals are as defined for formula I, the acyl radical can be removed by hydrolysis, yielding the corresponding compound of formula I wherein G is hydroxy-substituted lower alkylene. The hydrolysis is preferably carried out under customary conditions, for example in the presence of acids or bases, such as HCl or NaOH, in aqueous solution or in a suitable solvent or solvent mixture.

From a compound of formula I wherein G is acyloxy-substituted lower alkyl it is also possible to prepare a compound of formula I wherein G is lower alkylene. The reaction in that case is preferably carried out with catalytic hydrogenation (hydrogen in the presence of a suitable catalyst) in a customary solvent or solvent mixture.

### General process conditions

All the process steps mentioned in the present text can be carried out under reaction conditions which are known *per se,* preferably those mentioned specifically, in the absence or, customarily, in the presence of solvents or diluents, preferably those which are inert towards the reagents used and are solvents therefor, in the absence or presence of catalysts, condensing agents or neutralising agents, for example ion exchangers, such as cation exchangers, for example in the H⁺ form, depending on the nature of the reaction and/or of the reactants at reduced, normal or elevated temperature, for example in a temperature range of from approximately -100°C to approximately 190°C, preferably from approximately -80°C to approximately 150°C, for example at from -80 to -60°C, at room temperature, at from -20 to 40°C or at the boiling point of the solvent used, under atmospheric pressure or in a closed vessel, where appropriate under pressure, and/or in an inert atmosphere, for example under an argon or nitrogen atmosphere.

In all starting materials and intermediate compounds, salts can be present where salt-forming groups are present. Salts can also be present during the reaction of such compounds, provided that the reaction is not impaired thereby.

At all stages of the reaction, isomeric mixtures that form can be separated into the individual isomers, for example diastereoisomers or enantiomers, or into any desired mixtures of isomers, for example racemates or diastereoisomeric mixtures, for example analogously to the methods described under "Additional process steps".

In certain cases, for example in the case of hydrogenations, it is possible to achieve stereoselective reactions so that, for example, it is easier to obtain individual isomers.

The solvents from which those that are suitable for a particular reaction can be selected include, for example, water, esters, such as lower alkyl lower alkanoates, for example diethyl acetate, ethers, such as aliphatic ethers, for example diethyl ether, or cyclic ethers, for example tetrahydrofuran, liquid aromatic hydrocarbons, such as benzene or toluene, alcohols, such as methanol, ethanol or 1- or 2-propanol, nitriles, such as acetonitrile, halogenated hydrocarbons, such as methylene chloride, acid amides, such as dimethylformamide, bases, such as heterocyclic nitrogen bases, for example pyridine, carboxylic acids, such as lower alkanecarboxylic acids, for example acetic acid, carboxylic acid anhydrides, such as lower alkanoic acid anhydrides, for example acetic anhydride, cyclic, linear or branched hydrocar bons, such as cyclohexane, hexane or isopentane, or mixtures of those solvents, for example aqueous solutions, unless indicated otherwise in the description of the processes. Such solvent mixtures can also be used in working up, for example by chromatography or partitioning.

The invention relates also to those forms of the process in which a compound obtainable as an intermediate at any stage is used as starting material and the remaining steps are carried out, or the process is interrupted at any stage, or a starting material is formed under the reaction conditions or is used in the form of a reactive derivative or salt, or a compound obtainable by the process according to the invention is produced under the process conditions and is processed further *in situ.* There are preferably used those starting materials which lead to the compounds described above as being preferred, especially as being especially preferred, more especially preferred and/or very especially preferred.

The preparation of compounds of formula I (or N-oxides thereof) is preferably carried out analogously to the processes and process steps mentioned in the Examples.

The compounds of formula I (or N-oxides thereof), including their salts, can also be obtained in the form of hydrates, or their crystals can include, for example, the solvent used for crystallisation (presence in the form of solvates).

### Pharmaceutical compositions and uses

The present invention relates also to pharmaceutical compositions which comprise a compound of formula I (or an N-oxide thereof) as active ingredient and can be used especially in the treatment of the disease mentioned at the beginning. Special preference is given to compositions for enteral, such as nasal, buccal, rectal or, especially, oral, and parenteral, such as intravenous, intramuscular or subcutaneous, administration to warm-blooded animals, especially human beings. The compositions comprise the active ingredient on its own or, preferably, together with a pharmaceutically acceptable carrier. The dose of active ingredient depends on the disease to be treated and on the species, its age, weight and individual condition, individual pharmacokinetic data and on the mode of administration.

The invention relates also to pharmaceutical compositions for use in a method of treating the human or animal body prophylactically or, especially, therapeutically, according to the claims in the form of compositions for the treatment of melanoma and to the use for treating the claimed diseases,

Preference is given to a pharmaceutical composition which is suitable for administration to a warm-blooded animal, especially a human being or a commercially useful mammal, which is suffering from the disease characterized by an aberrant MAP kinase signaling pathway melanoma, comprising a compound of formula I (or an N-oxide thereof), or a pharmaceutically acceptable salt thereof where salt-forming groups are present, in an amount that is effective in inhibiting RAF kinase, particularly a mutant RAF kinase, together with at least one pharmaceutically acceptable carrier.

Preference is given also to a pharmaceutical composition for the prophylactic or, especially, therapeutic treatment of melanoma in a warm-blooded animal, especially a human being or a commercially useful mammal, which requires such treatment, especially which is suffering from such a disease, comprising a novel compound of formula I (or an N-oxide thereof), or a pharmaceutically acceptable salt thereof, as active ingredient in an amount that is effective prophylactically or, especially, therapeutically against the mentioned diseases.

Pharmaceutical compositions comprise from approximately I % to approximately 95 % active ingredient, dosage forms that are in single dose form preferably comprising from approximately 20 % to approximately 90 % active ingredient, and dosage forms that are not in single dose form preferably comprising from approximately 5 % to approximately 20 % active ingredient. Unit dose forms are, for example, dragées, tablets, ampoules, vials, suppositories or capsules. Other dosage forms are, for example, ointments, creams, pastes, foams, tinctures, lipsticks, drops, sprays, dispersions, etc.. Examples are capsules comprising from approximately 0.05 g to approximately 1.0 g of the active ingredient.

The pharmaceutical compositions of the present invention are prepared in a manner known *per se,* for example by means of conventional mixing, granulating, confectioning, dissolving or lyophilising processes.

Solutions of the active ingredient are preferably used, in addition also suspensions or dispersions, especially isotonic aqueous solutions, dispersions or suspensions, which, in the case of, for example, lyophilised compositions which contain the active substance alone or together with a carrier, for example mannitol, can be prepared prior to use. The pharmaceutical compositions may be sterilised and/or comprise excipients, for example preservatives, stabilisers, wetting agents and/or emulsifiers, solubilisers, salts for regulating the osmotic pressure and/or buffers, and are prepared in a manner known *per se,* for example by means of conventional dissolving or lyophilising processes. The mentioned solutions or suspensions may comprise viscosity-increasing substances, such as sodium carboxymethylcellulose, carboxymethylcellulose, dextran, polyvinylpyrrolidone or gelatin, or solubilisers, for example Tween 80 [polyoxyethylene(20)sorbitan monooleate; trade mark of ICI Americas, Inc, USA].

Suspensions in oil comprise as the oily component the vegetable, synthetic or semi-synthetic oils customary for injection purposes. There may be mentioned as such especially liquid fatty acid esters, which comprise as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid or linoleic acid, optionally with the addition of antioxidants, for example vitamin E, β-carotene or 3,5-di-tert-butyl-4-hydroxytoluene. The alcohol component of those fatty acid esters has a maximum of 6 carbon atoms and is a mono- or poly-hydric, for example mono-, di- or tri-hydric, alcohol, for example methanol, ethanol, propanol, butanol or pentanol or their isomers, but especially glycol and glycerol. Examples of fatty acid esters which may be mentioned are, therefore: ethyl oleate, isopropyl myristate, isopropyl palmitate, "Labrafil M 2375" (polyoxyethyleneglycerol trioleate from Gattefossé, Paris), "Labrafil M 1944 CS" (unsaturated polyglycolised glycerides prepared by alcoholysis of apricot kernel oil and composed of glycerides and polyethylene glycol ester; Gattefossé, France), "Labrasol" (saturated polyglycolised glycerides prepared by alcoholysis of TCM and composed of glycerides and polyethylene glycol ester; Gattefossé, France) and/or "Miglyol 812" (triglyceride of saturated fatty acids having a chain length of from C₈ to C₁₂ from Hüls AG, Germany), but especially vegetable oils, such as cottonseed oil, almond oil, olive oil, castor oil, sesame oil, soybean oil and, more especially, groundnut oil.

The preparation of the injection compositions is carried out in customary manner under sterile conditions, as are also the introduction thereof, for example, into ampoules or vials and the sealing of the containers.

Pharmaceutical compositions for oral administration can be obtained, for example, by combining the active ingredient with one or more solid carriers, granulating a resulting mixture, where appropriate, and processing the mixture or granules, if desired, where appropriate by addition of additional excipients, to tablets or dragée cores.

Suitable carriers are especially fillers, such as sugars, for example lactose, saccharose, mannitol or sorbitol, cellulose preparations and/or calcium phosphates, for example tricalcium phosphate or calcium hydrogen phosphate, also binders, such as starches, for example corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Dragée cores can be provided with suitable, optionally enteric, coatings, there being used *inter alia* concentrated sugar solutions which may contain gum arabic, talc, polyvinylpyrrolidone, polyethylene glycol and/or titanium dioxide, or coating solutions in suitable organic solvents or solvent mixtures or, for the preparation of enteric coatings, solutions of suitable cellulose preparations, such as acetylcellulose phthalate or hydroxypropylmethylcellulose phthalate. Colourings or pigments may be added to the tablets or dragée coatings, for example for identification purposes or to indicate different doses of active ingredient.

Pharmaceutical compositions for oral administration are also hard gelatin capsules and soft sealed capsules consisting of gelatin and a plasticiser, such as glycerol or sorbitol. The hard gelatin capsules may contain the active ingredient in the form of granules, for example in admixture with fillers, such as corn starch, binders and/or glidants, such as talc or magnesium stearate, and optionally stabilisers. In soft capsules the active ingredient is preferably dissolved or suspended in suitable liquid excipients, such as fatty oils, paraffin oil or liquid polyethylene glycols or fatty acid esters of ethylene glycol or propylene glycol, it likewise being possible to add stabilisers and detergents, for example of the polyoxyethylenesorbitan fatty acid ester type.

Suitable rectally administrable pharmaceutical compositions are, for example, suppositories that consist of a combination of the active ingredient with a suppository base. Suitable suppository bases are, for example, natural or synthetic triglycerides, paraffin hydrocarbons, polyethylene glycols or higher alkanols.

For parenteral administration there are suitable, especially, aqueous solutions of an active ingredient in water-soluble form, for example in the form of a water-soluble salt, or aqueous injection suspensions that comprise viscosity-increasing substances, for example sodium carboxymethylcellulose, sorbitol and/or dextran and, if desired, stabilisers. The active ingredient, optionally together with excipients, can also be in the form of a lyophilisate and can be made into a solution prior to parenteral administration by the addition of suitable solvents.

Solutions used, for example, for parenteral administration can also be used as infusion solutions.

Preferred preservatives are, for example, antioxidants, such as ascorbic acid, or microbicides, such sorbic acid or benzoic acid.

The invention relates especially to the use for treating one of the pathological conditions that is characterized by an aberrant MAP kinase signaling pathway, especially a disease responsive to inhibition of RAF kinase, namely melanoma. The compounds of formula I (or an N-oxide thereof) can be administered prophylactically or therapeutically as such or in the form of pharmaceutical compositions, preferably in an amount that is effective against the mentioned diseases, to a warm-blooded animal, for example a human being, requiring such treatment, the compounds being used especially in the form of pharmaceutical compositions. In the case of a body weight of approximately 70 kg, a daily dose of from approximately 0.1 g to approximately 5 g, preferably from approximately 0.5 g to approximately 2 g, of a compound of the present invention is administered.

The preferred dosage, composition and preparation of pharmaceutical formulations (medicaments) to be used in each particular case are described above.

### Starting materials

The starting materials used and the reaction conditions chosen are preferably such that the compounds mentioned as being preferred are obtained.

The starting materials of formulae II and IIII are known, can be prepared by processes known *per* se, or are available commercially; in particular, they can be prepared by processes analogous to those mentioned in the Examples.

In the preparation of starting materials, any functional groups present that are not to take part in the reaction may be in protected form, if necessary. Preferred protecting groups, their introduction and their removal are described under process a) or in the Examples. Instead of the starting materials and intermediates in question, it is also possible to react salts thereof where salt-forming groups are present and the reaction in question is also possible using a salt. Therefore, any reference hereinbefore and hereinafter to starting materials is also intended to include their salts, where expedient and possible.

Compounds of formula II wherein G is -CH₂-O-, -CH₂-S-, -CH₂-NH-, oxa, thia or NR- and the other symbols are as defined for formula I can be prepared, for example, by reacting a compound of formula IV 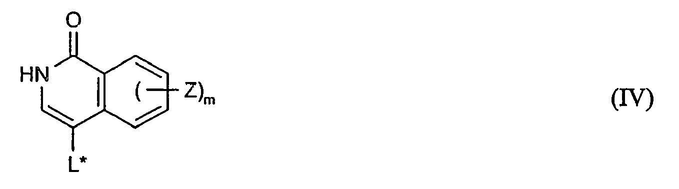 wherein L* is a nucleofugal leaving group, especially halo, such as bromo, and m and Z and the bonds indicated by wavy lines are as defined for a compound of formula I (especially m = 0, i.e. Z is not present - the corresponding compound of formula IV wherein L* is bromo is available commercially from SPECS & BIOSPECS, Rijskwijk, Holland), with a compound of formula V 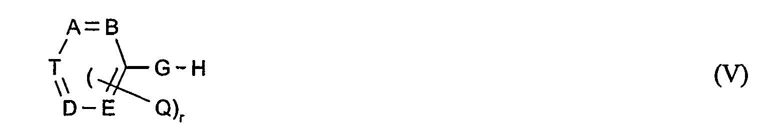 wherein G is -CH₂-O-, -CH₂-S- or -CH₂-NH-, or is oxa, thia or -NR-, and A, B, D, E, T, Q and r are as defined for compounds of formula I, preferably under conditions analogous to those mentioned under process a) for the reaction of a compound of formula II with a compound of formula III, or with palladium complex catalysis with Pd⁰, for example with tetrakis(triphenylphosphinyl)palladium complexes, palladium(0)-P(o-tolyl)₃ complexes, palladium(0) complexes with chelating bis(phosphines) (see, for example, J. Org. Chem. 61, 7240-7241 (1996)) or the like, preferably with Pd⁰ in the presence of an alkali metal carbonate, such as K₂CO₃, in a suitable solvent, such as toluene, at elevated temperature, preferably under reflux. There is then obtained a compound of formula II* 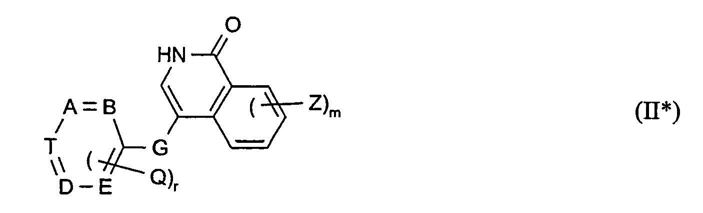 wherein m and Z and the bonds indicated by wavy lines, and A, B, D, E, T, Q and r are as defined for a compound of formula I, and wherein G is -CH₂-O-, -CH₂-S- or -CH₂-NH-, or is oxa, thia or NR-.

The corresponding compound of formula II can be prepared therefrom by introduction of a nucleofugal group L, as defined for formula II, using a corresponding acid anhydride, for example phosphoryl chloride (POCl₃) for the introduction of L = Cl or a different reagent mentioned below for the conversion of a compound of formula XII into a compound of formula II.

The starting materials of formulae IV and V are known, can be prepared by processes known *per se,* or are available commercially.

A compound of formula II wherein G is methylene and the other symbols are as defined for a compound of formula I can be prepared, for example, by reacting a lactone of formula VI 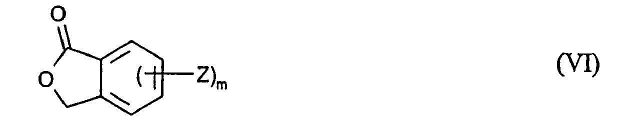 wherein Z and m are as defined for a compound of formula I, with an alkali metal cyanide, especially potassium cyanide, at elevated temperature, for example at from 100°C to 200°C (see Org. Synthesis, Coll. Vol. 3, 174), yielding a cyanomethylbenzoic acid of formula VII 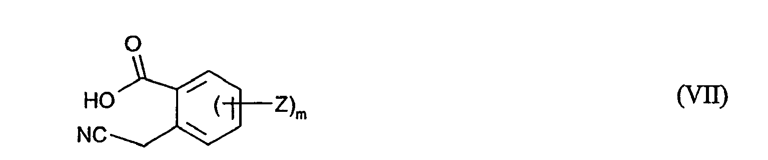 wherein the radicals are as defined for formula VI; the compound of formula VII is then converted into the lower alkyl ester, for example by adding a suitable di-lower alkylformamide di-lower alkylacetyl, such as dimethylformamide dimethylacetyl, to the compound of formula VII in a suitable solvent, for example a halo-lower alkane, such as dichloromethane, and stirring the mixture to complete the reaction, preferably at temperatures of from 0 to 60°C, for example at approximately room temperature. The corresponding lower alkyl ester of formula VIII is obtained 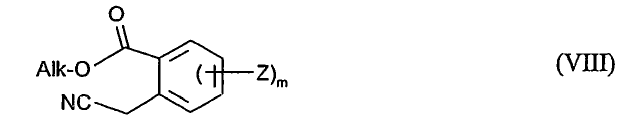 wherein Alk is lower alkyl, especially methyl, and the other radicals are as defined for formula VI. The ester is then reacted in a suitable solvent, for example an ether, such as tetrahydrofuran, or an ester, such as ethyl propionate, or mixtures thereof, with an aldehyde of formula IX 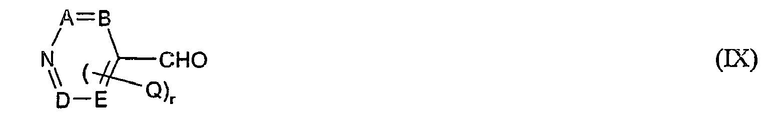 wherein A, B, D, E, Q and r are as defined for a compound of formula I, in the presence of an alcohol, such as methanol, and of the corresponding alcoholate, such as an alkali metal methanolate, for example sodium methanolate, at a temperature of preferably from 0°C to reflux temperature, preferably at approximately from 5 to 30°C, yielding the compound of formula X 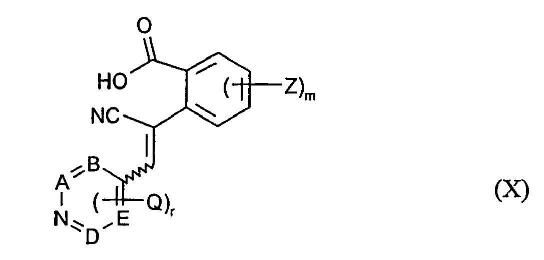 wherein the radicals A, B, D, E, Q and Z and the indices r and m are as defined for a compound of formula I; that compound is converted (under conditions analogous to those for the preparation of the lower alkyl ester of formula VIII) into the corresponding lower alkyl ester of formula XI 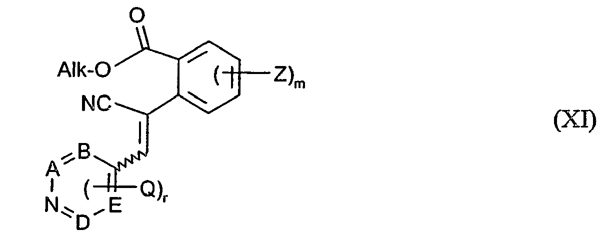 wherein Alk is lower alkyl, especially methyl, and the other radicals are as defined for formula X. Subsequent hydrogenation in the presence of a suitable catalyst, especially a framework catalyst, such as Raney cobalt or, especially, Raney nickel, in a suitable solvent, such as an ether, for example tetrahydrofuran, or an alcohol, such as methanol, or mixtures thereof, at preferred temperatures of from 10 to 80°C, at pressures of from 0.5 to 100 bar, especially approximately at normal pressure, yields an isoquinoline compound of formula XII 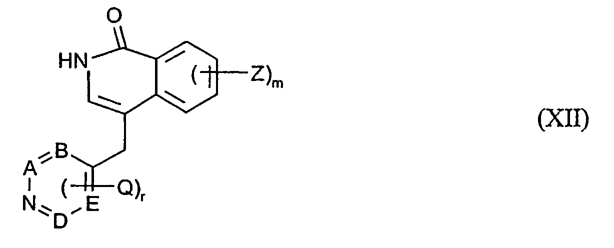 wherein the radicals A, B, D, E, Q and Z and the indices r and m are as defined for a compound of formula I.

The compound of formula XII is then converted into the corresponding compound of formula II, or a salt thereof, by means of a suitable reagent for the introduction of the nucleofugal leaving group, for example a phosphoryl halide or phosphorus pentahalide, especially phosphoryl chloride (POCl₃) or phosphorus pentachloride, without a solvent or in a suitable solvent, for example acetonitrile, in the absence or, preferably, in the presence of a corresponding acid, for example of a hydrohalic acid, such as hydrochloric acid, at preferred temperatures of from 40°C to reflux temperature, preferably at approximately from 40 to 60°C.

In an analogous manner, it is possible using compounds analogous to those of formula IX wherein, however, the place of the -CHO- group is taken by a corresponding lower alkanealdehyde group, *via* compounds analogous to those of formulae X to XII wherein the place of the methylidene group (formula X, XI) or of the methylene group (formula XII) is taken by a corresponding lower alkylidene or lower alkylene group, to prepare corresponding compounds of formula II wherein G is lower alkylene.

The other starting materials are known, can be prepared by processes known *per se,* or are available commercially, or, in particular, can be prepared by processes analogous to those mentioned in the Examples.

### The Examples which follow serve to illustrate the invention

### Synthesis Examples (Reference)

### Example S1: 1-(3,5-Dimethylanilino)-4-[(pyridin-4-yl)-methyl]-isoquinoline (= N-(3,5-dimethyl-phenyl)-[4-(pyridin-4-yl-methyl)-isoquinolin-1-yl]-amine)

With the exclusion of moisture, 100 mg (0.825 mmol) of 3,5-dimethyl-aniline are dissolved in 4 ml of ethanol, and 196 µl (0.784 mmol) of HCl (4N in dioxane) are added. After the addition of 200 mg (0.785 mmol) of 1-chloro-4-[(pyridin-4-yl)-methyl]-isoquinoline, the mixture is heated for 8 hours at 90°C. Concentration by evaporation is then carried out; the residue is taken up in 4 ml of water, 1 ml of saturated ammonia solution and 20 ml of CH₂Cl₂, and the organic phase is separated off, dried with Na₂SO₄ (anhydrous) and concentrated by evapo ration again. Column chromatography (SiO₂; ethyl acetate/hexane 3:1) and crystallisation from ethyl acetate/hexane yield the title compound: m.p. 156-158°C; FA-B-MS: (M+H)⁺=340; Anal. *calc.* (C₂₃H₂₁N₃ 0.1 H₂O) C 80.95 %, H 6.26 %, N 12.31 %; *found* C 80.9 %, H 6.2 %, N12.4%.

The starting material is prepared as follows:

### S1a) 2-Cyanomethyl-benzoic acid methyl ester

With gentle heating, 175 g (1.08 mol) of 2-cyanomethyl-benzoic acid (for preparation see: Org. Synthesis, Coll, Vol. 3, 174) are dissolved in 1.7 litres of CH₂Cl₂; 242 ml (≈90 %, 1.6 mol) of dimethylformamide dimethylacetal are added dropwise at room temperature, and stirring is carried out for 38 hours to complete the reaction. The reaction mixture is washed with 2 x 1.2 litres of saturated NaHCO₃ solution and brine. The aqueous phases are extracted using 2 portions of CH₂Cl₂, and the organic phases are dried (Na₂SO₄) and concentrated by evaporation. Column chromatography (SiO₂; ethyl acetate/hexane 1:4-, applied in ethyl acetate/hexane/CH₂Cl₂) yields the title compound: m.p. 49-50°C Anal. *calc.* (C₁₀H₉NO₂) C 68.56 %, H 5.18 %, N 8.00 %; *found* C 68.5 %, H 5.1 %, N 7.9 %.

### S1b) 2-[(1-Cyano-2-(pyridin-4-yl)-vinyl]-benzoic acid

With the exclusion of air, 127.7 ml (1.35 mol) of pyridine-4-carbaldehyde (Fluka, Buchs, Switzerland) are added to a solution of 215.6 g (1.23 mol) of 2-cyanomethyl-benzoic acid methyl ester in 1.8 litres of THF. The mixture is cooled to 8-9°C, 297 ml (1.6 mol) of a 5.4M solution of sodium methanolate in methanol are added dropwise in the course of 20 minutes, and the mixture is stirred for 1.5 hours at from 10 to 15°C. The mixture is then adjusted to pH 6.0 using approximately 350 ml of 4N HCl and is then stirred for one hour at 5°C. The title compound crystallises out and is filtered off with suction and washed thoroughly with THF/- water 2:1 and THF: m.p. 218-219°C; FAB-MS: (M+H)⁺=251;; Anal. *calc.* (C₁₅H₁₀N₂O₂) C 71.99%,H4.03%,N11.19%; *found* C71.9%, H4.1%, N11.1%.

### S1c) 2-[(1-Cyano-2-(pyridin-4-yl)-vinyl]-benzoic acid methyl ester

With the exclusion of moisture, 211 g (0.843 mol) of 2-[(1-cyano-2-(pyridin-4-yl)-vinyl]-benzoic acid are suspended in 3.3 litres of CH₂Cl₂; 169 ml (≈90 %, 1.1 mol) of dimethylformamide dimethylacetal (Fluka, Buchs, Switzerland) are added at room temperature, and stirring is carried out for 22 hours to complete the reaction. The reaction mixture is filtered, and the residue is washed thoroughly with CH₂Cl₂ and discarded. Concentration of the filtrate by evaporation, chromatography (SiO₂; ethyl acetate) and crystallisation from ethyl acetate/hexane yield the title compound: m.p. 102-104°C; FAB-MS: (M+H)⁺=265; Anal. *calc.* (C₁₆H₁₂N₂O₂) C 72.72 %, H 4.58 %, N 10.60 %; *found* C 72.7 %, H 4.8 %, N 10.5 %.

### S1d) 4-(Pyridin-4-yl-methyl)-2H-isoquinolin-1-one

In the presence of 5 x 40 g of Raney nickel (added at intervals), 163 g (617 mmol) of 2-[(1-cyano-2-(pyridin-4-yl)-vinyl]-benzoic acid methyl ester are hydrogenated in 3 litres of THF at 40°C for 90 hours. The reaction mixture is filtered, and the filtrate is concentrated by evaporation and crystallised from acetonitrile/ethyl acetate (→ title compound). Further product can be obtained from the mother liquor by chromatography (SiO₂; ethyl acetate-acetone): m.p. 189-190°C; FAB-MS: (M+H)⁺=237; Anal. *calc.* (C₁₅H₁₂N₂O . 0.05 H₂O) C 75.96 %, H 5.14 %, N 11.81 %; *found* C 75.8 %, H 5.2 %, N11.9 %.

### S1e) 1-Chloro-4-(pyridin-4-ylmethyl)-isoquinoline

With the exclusion of air, 32.7 g (139 mmol) of 4-(pyridin-4-yl-methyl)-2H-isoquinolin-1-one are made into a slurry in 560 ml of acetonitrile, and 69.2 ml (277 mmol) of 4N HCl in dio:cane and 31.7 ml (346 mmol) of POCl₃ are added. The mixture is stirred for 22 hours at 50°C and then cooled in an ice bath, and a solution of 128.6 g of NaHCO₃ in 1.64 litres of water is added in the course of 30 minutes. During the addition, first a clear solution forms and then the title compound precipitates and, after 15 minutes, can be filtered off, washed thoroughly with water and ether and dried under a high vacuum at 60°C: m.p. 119-120°C; FAB-MS: (M+H)⁺=255;.

### Example S2: 1-(3-Chlorobenzylamino)-4-[(pyridin-4-yl)-methyl]-isoquinoline

With the exclusion of moisture, 1.6 ml (13.1 mmol) of 3-chlorobenzylamine and 800 mg (3.14 mmol) of 1-chloro-4-(pyridin-4-ylmethyl)-isoquinoline (Example 1e) are stirred for 2 hours at 150°C. The mixture is suspended in ethyl acetate, 1 ml of concentrated ammonia solution is added, washing with water and brine is carried out, and the organic phase is dried (Na₂SO₄) and concentrated by evaporation. Column chromatography (SiO₂; ethyl acetate) yields the title compound: m.p. 141-142°C; FAB-MS: (M+H)⁺=360; Anal. *calc.* (C₂₂H₁₈N₃Cl) C 73.43 %, H 5.04 %, N 11.68 %, Cl 9.85 %; *found* C 73.2 %, H 5.1 %, N 11.6 %, Cl 9.9 %.

### Example S3: 1-(4-t-butylanilino)-4-[(pyridin-4-yl)-methyl]-isoquinoline

With the exclusion of moisture, 27.5ml (172.7 mmol) of 4-t-butyl-aniline and 7.0 g (27.5 mmol) of 1-chloro-4-(pyridin-4-ylmethyl)-isoquinoline (Example 1e) are stirred for 3 hours at 80°C under a nitrogen blanket. The mixture is then cooled and partitioned between 5% (w/v) NaHCO₃ (aq.) and ethyl acetate. The ethyl acetate phase is dried over MgSO₄ and evaporated. Flash chromatography (silica gel, 50% (v/v) ethyl acetate/hexane) followed by crystallization from hexane in a dry ice/isopropyl alcohol bath yields 8.6 g of a light yellow powder, m.p. 157-159°C; Anal. *calc.* C 81.71 %, H 6.86 %, N 11.43 %, %; *found* C 81.88 %, H 6.85 %, N 11.43 %.

### Biological Examples

Active B-Raf, C-Raf, and V599E B-Raf proteins of human sequence are purified from insect cells using the baculoviral expression system. Raf inhibition is tested in 96-well microplates coated with IκB-α and blocked with Superblock. The phosphorylation of IκB-α at Serine 36 is detected using a phospho-IκB-α specific antibody (Cell Signaling #9246), an anti-mouse IgG alkaline phosphatase conjugated secondary antibody (Pierce # 31320), and an alkaline phosphatase substrate, ATTOPHOS (Promega, #S101).

Each of the following compounds inhibits wild-type C-R.AF at an IC₅₀ of from 0.01 to 3.5 micromoles/liter and/or wild-type B-RAF at an IC₅₀ of from 0.03 to 3.7 micromoles/liter and/or mutant B-RAF (V599E) at an IC₅₀ of from 0.01 to 3.4 micromoles/liter.

| **Example No.** | | **Melting point (°C)** | **MS (M+H)⁺** |
|---|---|---|---|
| 1. | 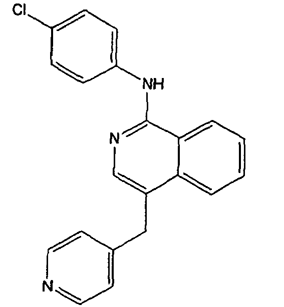 | 182-183 | 346 |
| 2. | 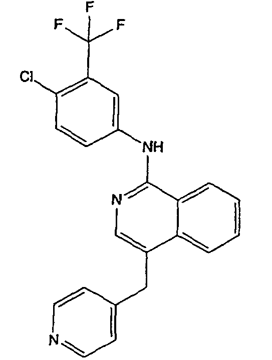 | 205-206 | 441 |
| 3. | 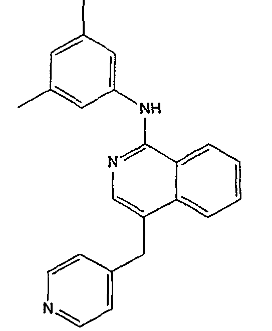 | 144-145 | 340 |
| 4. | 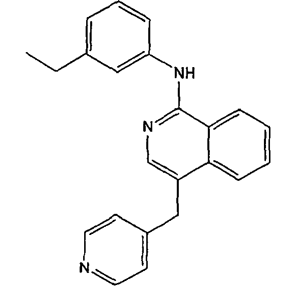 | 146-148 | 340 |
| 5. | 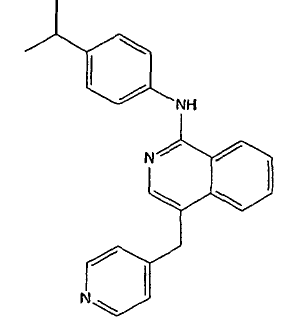 | 158-159 | 354 |
| 6. | 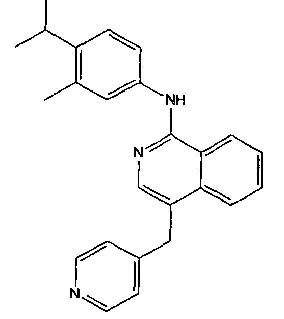 | | 368 |
| 7. | 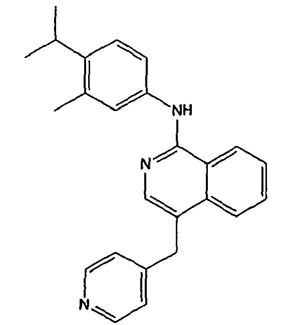 | | 368 |
| 8. | 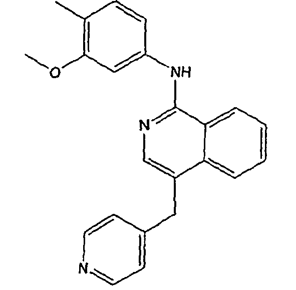 | | 356 |
| 9. | 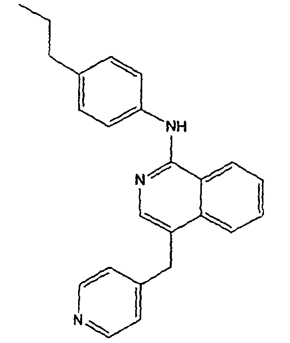 | 118-119 | 354 |
| 10. | 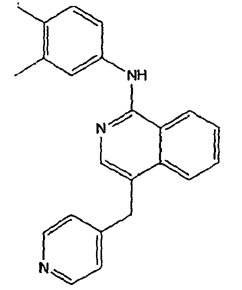 | 144-145 | 340 |
| 11. | 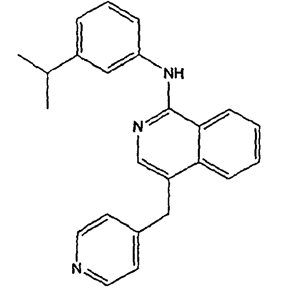 | 143-145 | 354 |
| 12. | 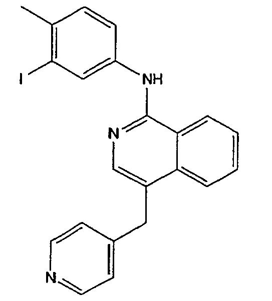 | 149-150 | 452 |
| 13. | 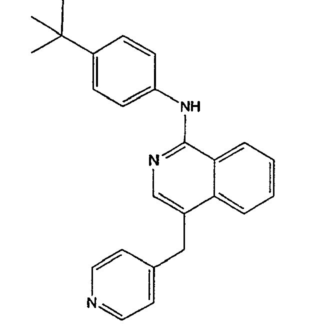 | | |
| 14. | 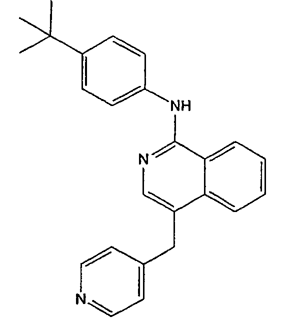 | 158-159 | 368 |
| 15. | 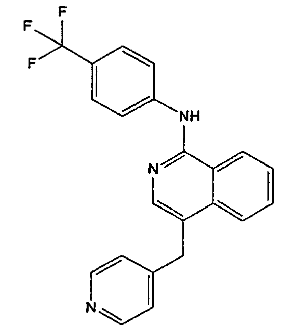 | 160-161 | 380 |
| 16. | 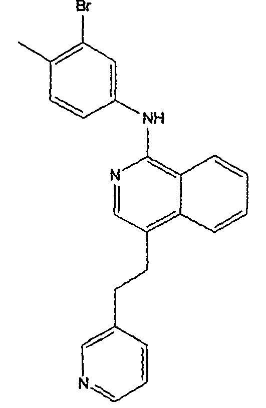 | 167-168 | 404/406 |
| 17. | 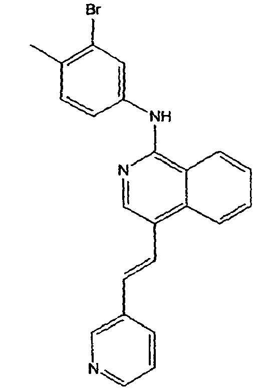 | | 416 |
| 18. | 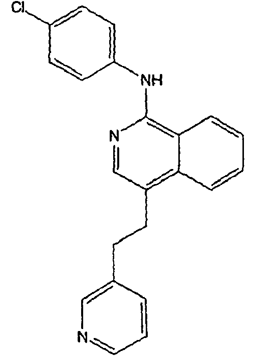 | | 359 |
| 19. | 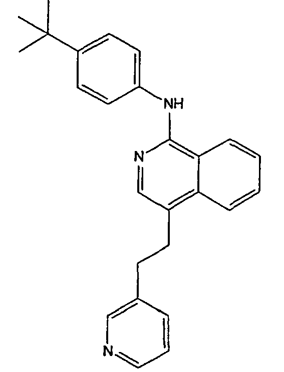 | 81 | |
| 20. | 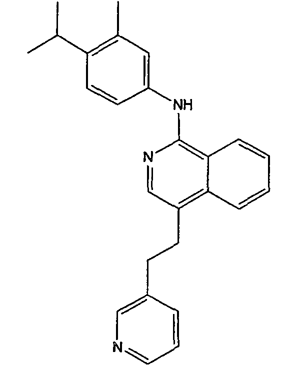 | 133-134 | |
| 21. | 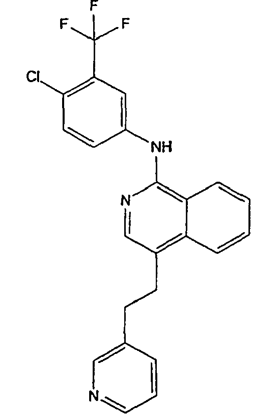 | | 159 |
| 22. | 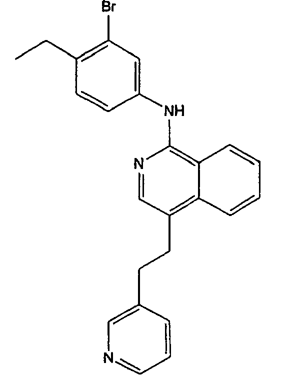 | | 119 |
| 23. | 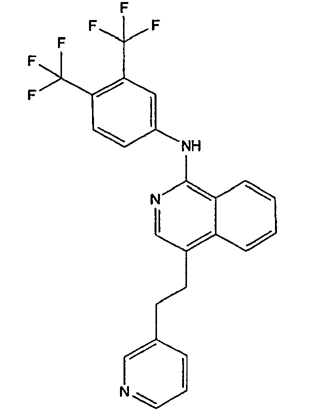 | 124-125 | |
| 24. | 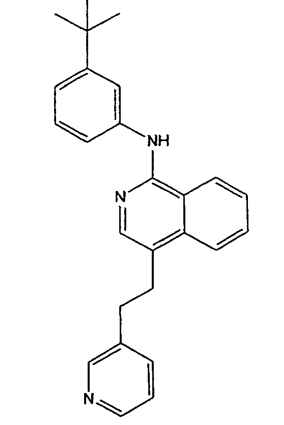 | 133-134 | |
| 25. | 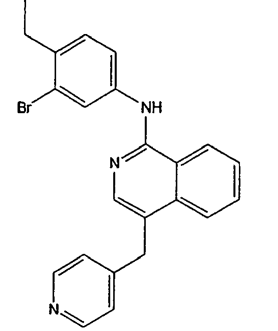 | | 418/420 |
| 26. | 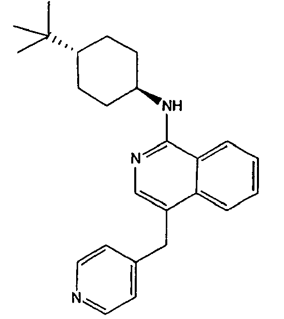 | | 374 |
| 27. | 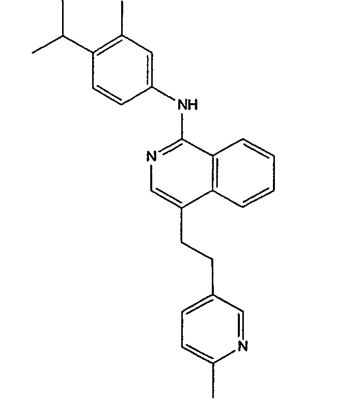 | | oil |
| 28. | 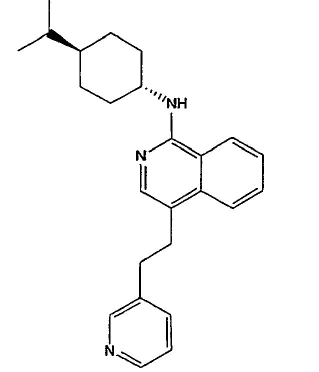 | 89-92 | |
| 29. | 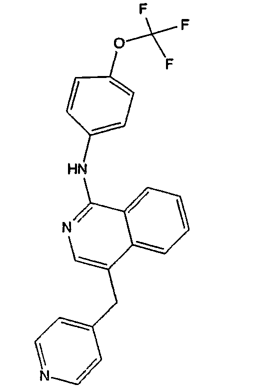 | | 396 |
| 30. | 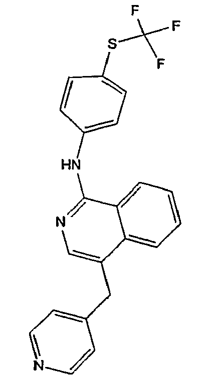 | | 412 |
| 31. | 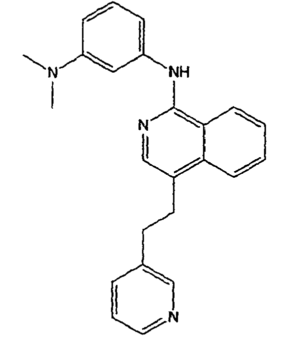 | | 369 |
| 32. | 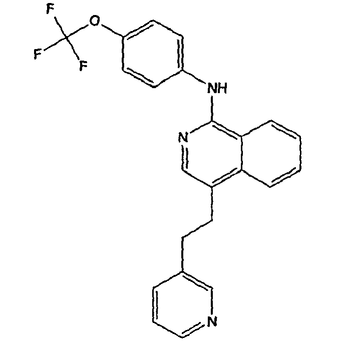 | | 410 |
| 33. | 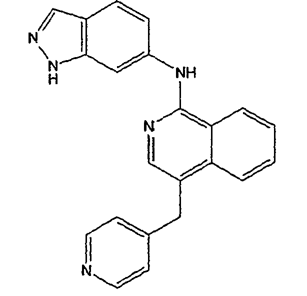 | | 332 |
| 34. | 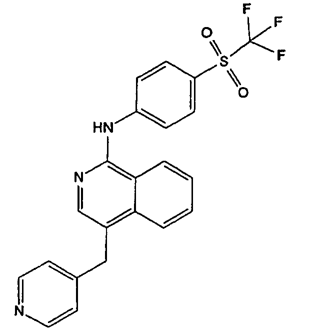 | | 444 |
| 35. | 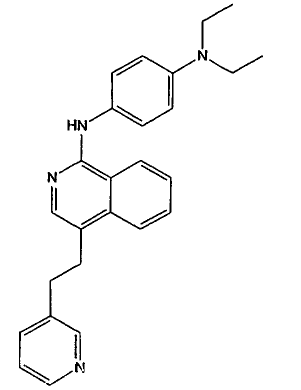 | | 397 |
| 36. | 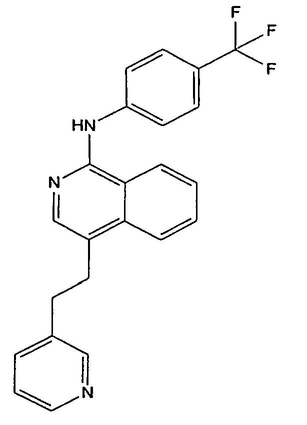 | | 394 |
| 37. | 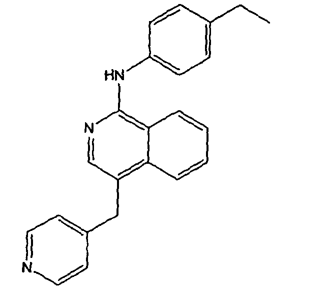 | | 340 |
| 38. | 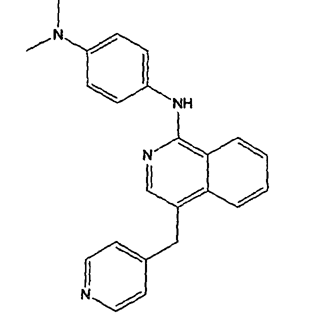 | | 355 |
| 39. | 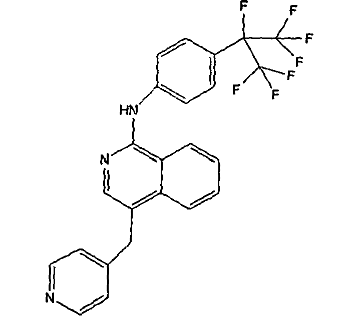 | | 480 |
| 40. | 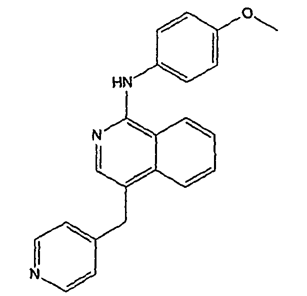 | | 342 |
| 41. | 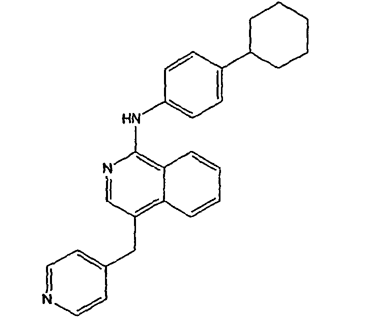 | | 394 |
| 42. | 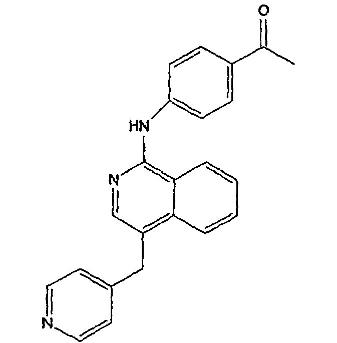 | | 354 |
| 43. | 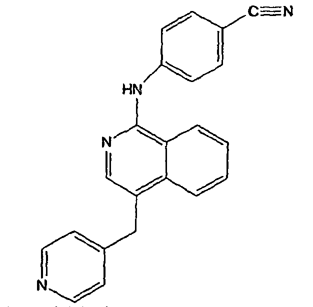 | | 337 |
| 44. | 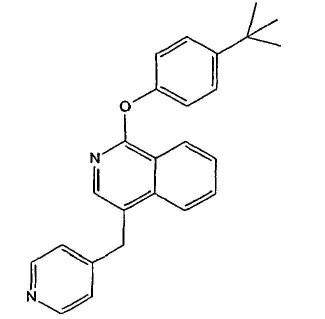 | | 369 |
| 45. | 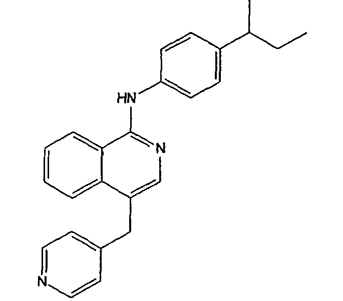 | | 368 |
| 46. | 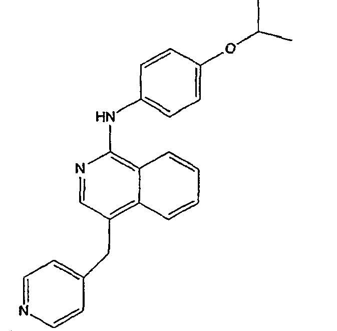 | | 370 |
| 47. | 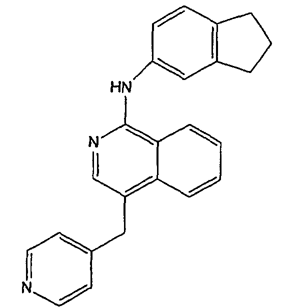 | | 352 |
| 48. | 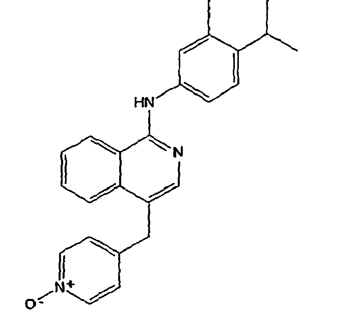 | | 384 |
| 49. | 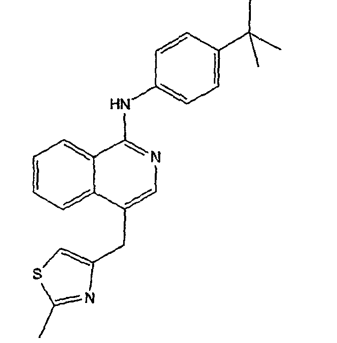 | | 388 |
| 50. | 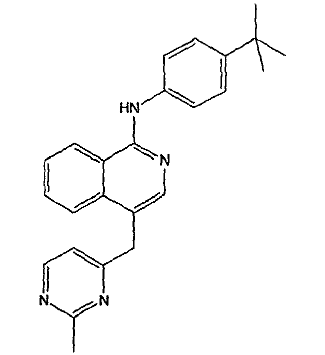 | | 383 |
| 51. | 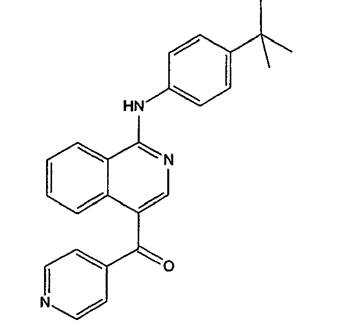 | | 382 |
| 52. | 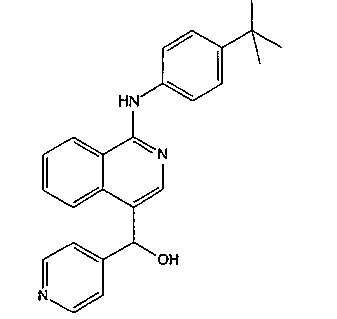 | | 384 |
| 53. | 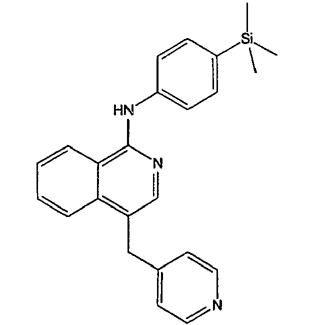 | | 384 |
| 54. | 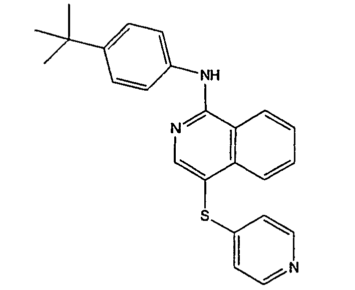 | | 386 |
| 55. | 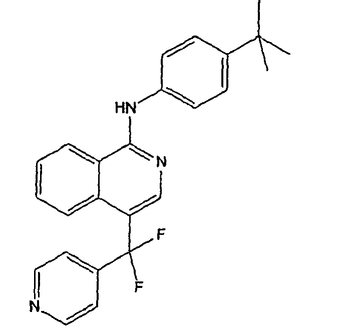 | | 404 |
| 56. | 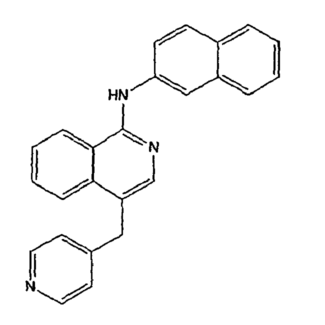 | | 362 |
| 57. | 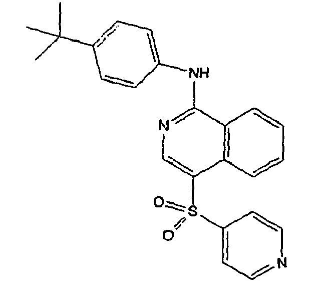 | | 418 |
| 58. | 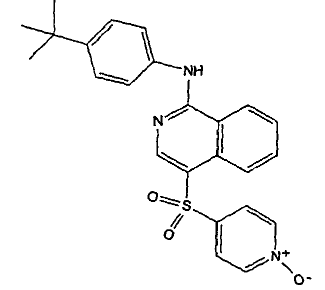 | | 434 |
| 59. | 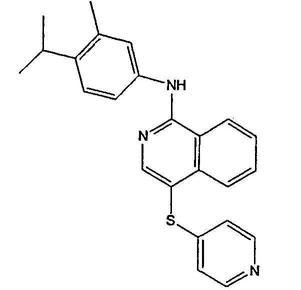 | | 386 |
| 60. | 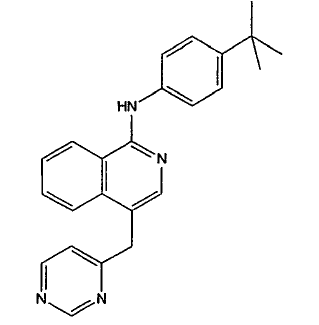 | | 369 |
| 61. | 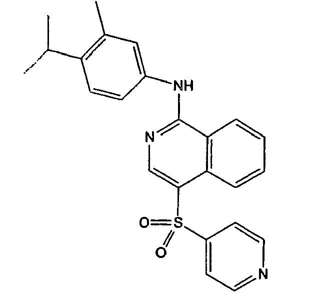 | | 418 |
| 62. | 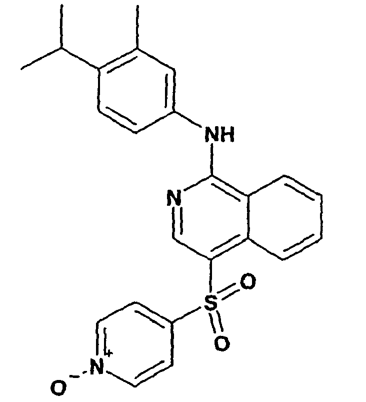 | | 433 |
| 63. | 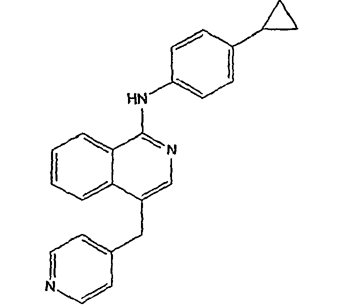 | | 351 |
| 64. | 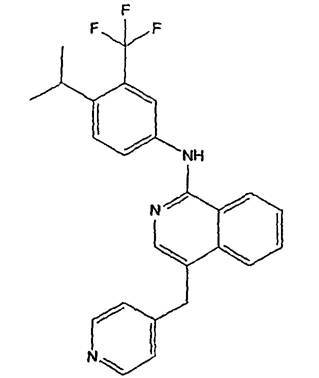 | | 422 |
| 65. | 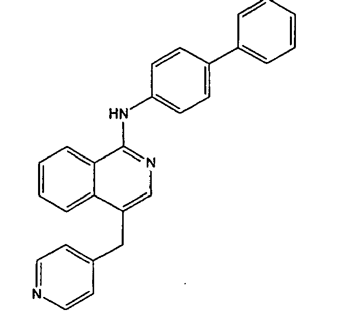 | | 387 |
| 66. | 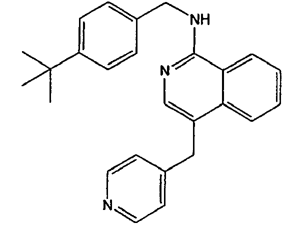 | | 382 |
| 67. | 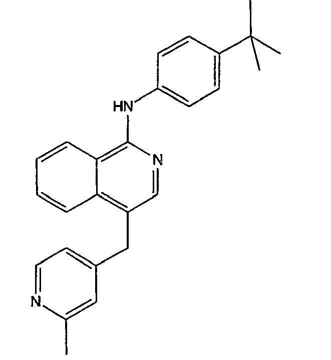 | | 382 |
| 68. | 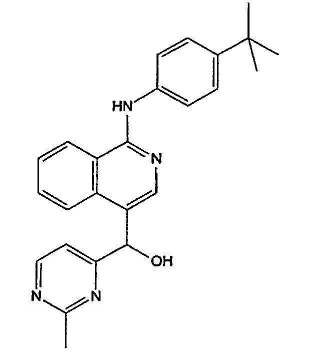 | | 399 |
| 69. | 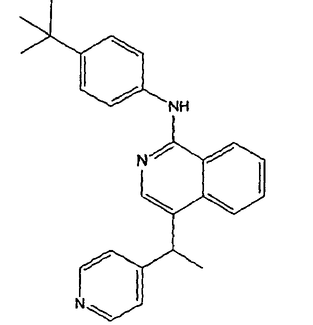 | | 382 |
| 70. | 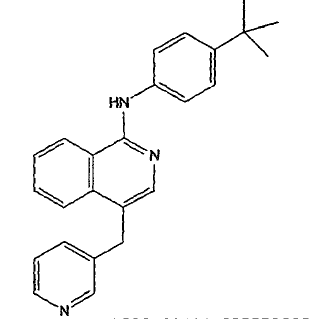 | | 368 |
| 71. | 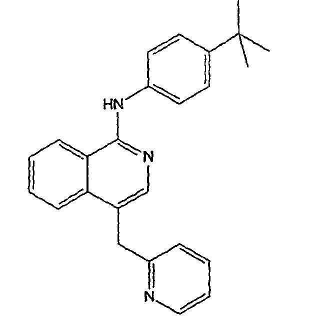 | | 368 |
| 72. | 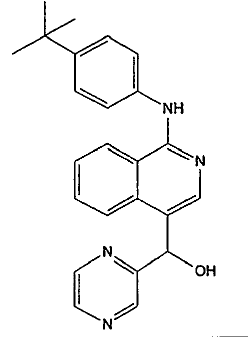 | | 384 |

### Example D1

### Detection of T1796A Mutation in the Human B-Raf Gene (Reference exemple)

Detection Primer: GATTTTGGTCTAGCTACAGA
Second Primer: GACTTTCTAGTAACTCAGCAG

Genomic DNA is isolated from human cells from a melanoma cell line using a GENELUTE mammalian genomic DNA kit (Sigma cat # G1 N 350). PCR reactions are carried out on a PCR machine (MJ Research, Model PTC100) in a total volume of 50 microL using the PCR Core kit by Roche (cat # 1578 553). The PCR reaction mixture contains 5 microL of 10X reaction buffer,1 microL of 10 mM dNTPs, 100-1000ng of template DNA, 0.5 microL Taq polymerase (2.5-5 units), 1 microL of a 31 uM stock of each primer.

The PCR conditions are as follows:
**95°C 3minutes**

| | |
|---|---|
| **94°C 1 minutes** | |
| **50°C 30 second** | **35** |
| | **Cycles** |
| **72°C 1 minutes** | |

**72°C 10minutes**
**4°C**

After amplification, 8 microLs of the PCR reaction mixture is mixed with 2 microLs of nucleic acid sample loading buffer [BioRad cat #161-0767]. The 10 microL sample is loaded onto a 1.5% agarose [GIBCO-BRL cat # 15510-027] gel that contains 0.3 ug/ml of ethidium bromide [Pierce cat #17898]. Molecular weight standards [100 bp DNA ladder from Invitrogen cat # 10380-012] are loaded in an adjacent lane. The DNA is separated by electrophoresis in TAE buffer (0.04 M Tris-acetate, 0.01 M EDTA. 0.02M glacial acetic acid pH8.4)[Roche cat #1666690]. Electrophoresis conditions are 120 volts for 30-40 minutes. After separation, the gel is exposed to UV light and a picture taken on a AlphaImager2000 documentation system.

Generally, two bands are detected in the gel. The faster migrating band runs ahead of the 100 bp marker and represents the primers. The DNA that results from the T1796A mutant specific PCR amplification has a predicted size of 152 bp and migrates between the 100 bp standard and the 200bp standard as predicted. The PCR amplification product is confirmed by sequencing. The presence of the PCR amplification product demonstrates that the T1796A mutation is present in the template DNA. The absence of the PCR amplification product is evidence that the mutation is absent in the tissue sample.

Other B-RAF mutations are detected by this method utilizing the detection primer and second primer indicated for the mutation in the following tables:

| **SEQ ID** | **Detection primer** | **B-RAF** |
|---|---|---|
| **NO:** | **oligonucleotide segment (5'→3')** | **mutation** |
| 1 | ACAGTGGGACAAAGAATTGA | G1388A |
| 2 | ACAGTGGGACAAAGAATTGT | G1388T |
| 3 | GGACAAAGAATTGGATCTGC | G1394C |
| 4 | GGACAAAGAATTGGATCTGA | G1394A |
| 5 | GGACAAAGAATTGGATCTGT | G1394T |
| 6 | ATTGGATCTGGATCATTTGC | G1403C |
| 7 | ATTGGATCTGGATCATTTGA | G1403A |
| 8 | GAGTAATAATATATTTCTTCATA | G1753A |
| 9 | CAGTAAAAATAGGTGATTTG | T1782G |
| 10 | CAGTAAAAATAGGTGATTTTC | G1783C |
| 11 | GTAAAAATAGGTGATTTTGGTG | C1786G |
| 12 | GTAAAAATAGGTGATTTTGGTCG | T1787G |
| 13 | GATTTTGGTCTAGCTACAGA | T1796A |
| 14 | GATTTTGGTCTAGCTACAGAT | TG1796-97AT |
| 15 | TGTCACCACATTACATACTTACC | G1388A |
| 16 | TGTCACCACATTACATACTTACC | G1388T |
| 17 | TGTCACCACATTACATACTTACC | G1394C |
| 18 | TGTCACCACATTACATACTTACC | G1394A |
| 19 | TGTCACCACATTACATACTTACC | G1394T |
| 20 | TGTCACCACATTACATACTTACC | G1403C |
| 21 | TGTCACCACATTACATACTTACC | G1403A |
| 22 | GACTTTCTAGTAACTCAGCAG | G1753A |
| 23 | GACTTTCTAGTAACTCAGCAG | T1782G |
| 24 | GACTTTCTAGTAACTCAGCAG | G1783C |
| 25 | GACTTTCTAGTAACTCAGCAG | C1786G |
| 26 | GACTTTCTAGTAACTCAGCAG | T1787G |
| 27 | GACTTTCTAGTAACTCAGCAG | T1796A |
| 28 | GACTTTCTAGTAACTCAGCAG | TG1796-97AT |

These examples are intended to further describe, this invention.

## Claims

1. Use of a compound of formula (I) 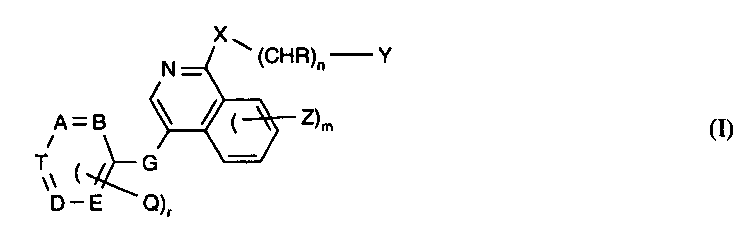 wherein
r is from 0 to 2;
n is from 0 to 2;
m is from 0 to 4;
A, B, D, E and T are each independently of the others N or CH, with the proviso that at least one, but not more than three, of A, B, D, E and T are N;
G is C₁ to C₇ alkylene, -CH₂-O-, -CH₂-S-, -CH₂-NH-, -SO₂-, oxa (-O-), thia (-S-) or -NR-, or C₁₋₇ alkylene substituted by acyloxy, oxo, halogen or hydroxy.
Q is C₁ to C₇ alkyl, especially methyl;
R is H or C₁ to C₇ alkyl;
X is Y, -N(R)-, oxa or thio; preferably -NH-;
Y is H, unsubstituted or substituted C₁ to C₇ alkyl, aryl, heteroaryl or unsubstituted or substituted cycloalkyl; and
Z is amino, mono- or di-substituted amino, halogen, alkyl, substituted alkyl, hydroxy, etherified or esterified hydroxy, nitro, cyano, carboxy, esterified carboxy, alkanoyl, carbamoyl, N-mono- or N,N-di-substituted carbamoyl, amidino, guanidino, mercapto, sulfo, phenylthio, phenyl- C₁ to C₇ alkylthio, alkylphenylthio, phenylsulfinyl, phenyl- C₁ to C₇ alkylsulfinyl, alkylphenylsulfinyl, phenylsulfonyl, phenyl- C₁ to C₇ alkanesulfonyl or alkylphenylsulfonyl, and where, if more than one radical Z is present (m ≥ 2), the substituents Z are identical or different;
or an N-oxide or a pharmaceutically acceptable salt thereof for the preparation of a medicament for the treatment of the disease **characterized by** excessive signaling through the MAP kinase signaling pathway Melanoma provided that the compound of formula (1) is not the compound wherein A, B, D, E, are CH, T is N, G is methylene, X is NH, Y is para - CL-phenyl.

2. A use according to claim 1 respectively wherein
r is from 0 to 2;
n is 0 or 1;
m is 0 or 1;
A, B, D and E are each CH and T is N, or A, D and E are each CH and B and T are N, or A, B, E and T are CH and D is N, or A, T, D and E are CH and B is N; particularly wherein A, B, D and E are each CH and T is N, or A, D and E are each CH and B and T are N.
G is C₁ to C₇ alkylene, -CH₂-NH-, -CH₂-O-, hydroxymethylene or benzoyloxy-methylene;
Q is methyl;
R is H or C₁ to C₇ alkyl;
X is -NR-, oxa or thia;
Y is phenyl that is unsubstituted or substituted by one or two identical or different substituents selected from the group consisting of amino; C₁ to C₇ alkanoylamino; halogen; unsubstituted or substituted C₁ to C₇ alkyl; hydroxy; C₁ to C₇ alkoxy; phenyl- C₁ to C₇ alkoxy; cyano, C₁ to C₇ alkenyl, C₈-C₁₂alkoxy, C₁ to C₇ alkoxycarbonyl, carbamoyl, C₁ to C₇ alkylcarbamoyl, C₁ to C₇ alkanoyl, phenyloxy, halo- C₁ to C₇ alkyloxy, C₁ to C₇ alkoxycarbonyl, C₁ to C₇ alkylmercapto, halo- C₁ to C₇ alkylmercapto, hydroxy- C₁ to C₇ alkyl, C₁ to C₇ alkanesulfonyl, halo- C₁ to C₇ alkanesulfonyl, phenylsulfonyl and C₁ to C₇ alkylenedioxy bonded to two adjacent carbon atoms or wherein two adjacent positions are substituted by alkylene or alkenylene to form a 5 to 7 membered ring that is fused to the phenyl ring;
Z is amino; N- C₁ to C₇ alkylamino; hydroxy- C₁ to C₇ alkylamino; phenyl- C₁ to C₇ alkylamino; N,N-di- C₁ to C₇ alkylamino; N-phenyl- C₁ to C₇ alkyl-N- C₁ to C₇ alkylamino; N,N-di- C₁ to C₇ alkylphenylamino; C₁ to C₇ alkanoylamino; benzoylamino and phenyl- C₁ to C₇ alkoxycarbonylamino, wherein the phenyl radical in each case is unsubstituted or substituted by nitro, amino, halogen, amino, N- C₁ to C₇ alkylamino, N,N-di- C₁ to C₇ alkylamino, hydroxy, cyano, carboxy, C₁ to C₇ alkoxycarbonyl, C₁ to C₇ alkanoyl, carbamoyl, 2-hydroxyethylamino, benzyloxycarbonylamino or bromine.

3. A use according to claim 1 respectively wherein
r is 0;
n is 0;
m is 0;
B, D, E and T are CH and A is N or A, B, D and E are each CH and T is N;
G is C₁₋₇ alkylene;
X is -NH-;
Y is phenyl that is unsubstituted or substituted by one or two identical or different substituents selected from the group consisting of halogen and C₁ to C₇ alkyl.

4. A use according to claim 1 respectively wherein
r is 0;
n is from 0 to 2;
m is 0;
A, B, D and E are each CH and T is N;
G is methylene;
R is H;
X is -NR; and
Y is phenyl that is unsubstituted or substituted by halogen, C₁ to C₇ alkyl, C₁ to C₇ alkoxy or cyclohexyl that is unsubstituted or substituted by C₁ to C₇ alkyl.

5. A use according to claim 1 respectively wherein
r is 0;
n is 0;
m is 0;
A, B, D and E are each CH and T is N;
G is methylene;
X -NH-; and
Y is phenyl that is substituted by one or two identical or different substituents selected from halogen and C₁ to C₇ alkyl.

6. A use according to claim 5 wherein Y is phenyl that is substituted in the 4-position by t-butyl.

7. A use according to claim 5 wherein Y is phenyl that is substituted in the 4-position by trifluoromethyl.

8. Use of a compound of claim 1 wherein the melanoma expresses mutant RAF kinase or over expresses a wild - type RAF kinase.

9. A use according to claim 8 respectively wherein
r is from 0 to 2;
n is 0 or 1;
m is 0 or 1;
A, B, D and E are each CH and T is N, or A, D and E are each CH and B and T are N, or A, B, E and T are CH and D is N, or A, T, D and E are CH and B is N; particularly wherein A, B, D and E are each CH and T is N, or A, D and E are each CH and B and T are N.
G is C₁ to C₇ alkylene, -CH₂-NH-, -CH₂-O-, hydroxymethylene or benzoyloxy-methylene;
Q is methyl;
R is H or C₁ to C₇ alkyl;
X is -NR-, oxa or thia;
Y is phenyl that is unsubstituted or substituted by one or two identical or different substituents selected from the group consisting of amino; C₁ to C₇ alkanoylamino; halogen; unsubstituted or substituted C₁ to C₇ alkyl; hydroxy; C₁ to C₇ alkoxy; phenyl- C₁ to C₇ alkoxy; cyano, C₁ to C₇ alkenyl, C₈-C₁₂alkoxy, C₁ to C₇ alkoxycarbonyl, carbamoyl, C₁ to C₇ alkylcarbamoyl, C₁ to C₇ alkanoyl, phenyloxy, halo- C₁ to C₇ alkyloxy, C₁ to C₇ alkoxycarbonyl, C₁ to C₇ alkylmercapto, halo- C₁ to C₇ alkylmercapto, hydroxy- C₁ to C₇ alkyl, C₁ to C₇ alkanesulfonyl, halo- C₁ to C₇ alkanesulfonyl, phenylsulfonyl and C₁ to C₇ alkylenedioxy bonded to two adjacent carbon atoms or wherein two adjacent positions are substituted by alkylene or alkenylene to form a 5 to 7 membered ring that is fused to the phenyl ring;
Z is amino; N- C₁ to C₇ alkylamino; hydroxy- C₁ to C₇ alkylamino; phenyl- C₁ to C₇ alkylamino; N,N-di- C₁ to C₇ alkylamino; N-phenyl- C₁ to C₇ alkyl-N- C₁ to C₇ alkylamino; N,N-di- C₁ to C₇ alkylphenylamino; C₁ to C₇ alkanoylamino; benzoylamino and phenyl- C₁ to C₇ alkoxycarbonylamino, wherein the phenyl radical in each case is unsubstituted or substituted by nitro, amino, halogen, amino, N- C₁ to C₇ alkylamino, N,N-di- C₁ to C₇ alkylamino, hydroxy, cyano, carboxy, C₁ to C₇ alkoxycarbonyl, C₁ to C₇ alkanoyl, carbamoyl, 2-hydroxyethylamino, benzyloxycarbonylamino or bromine.

10. A use according to claim 8 respectively wherein
r is 0;
n is 0;
m is 0;
B, D, E and T are CH and A is N or A, B, D and E are each CH and T is N;
G is C₁ to C₇ alkylene;
X is -NH-;
Y is phenyl that is unsubstituted or substituted by one or two identical or different substituents selected from the group consisting of halogen and C₁ to C₇ alkyl.

11. A use according to claim 8 respectively wherein
r is 0;
n is from 0 to 2;
m is 0;
A, B, D and E are each CH and T is N;
G is methylene;
R is H;
X is -NR; and
Y is phenyl that is unsubstituted or substituted by halogen, C₁ to C₇ alkyl, C₁ to C₇ alkoxy or cyclohexyl that is unsubstituted or substituted by lower alkyl.

12. A use according to claim 8 respectively wherein
r is 0;
n is 0;
m is 0;
A, B, D and E are each CH and T is N;
G is methylene;
X -NH-; and
Y is phenyl that is substituted by one or two identical or different substituents selected from halogen and C₁ to C₇ alkyl.

13. A use according to claim 12 wherein Y is phenyl that is substituted in the 4-position by t-butyl.

14. A use according to claim 8 respectively wherein the melanoma expresses a mutant RAF kinase.

15. A use according to claim 14 wherein the mutant RAF kinase corresponds to a mutation in the B-RAF kinase gene selected from G1388A, G1388T, G1394C, G1394A, G1394T, G1403C, G1403A, G1753A, T1782G, G1783C, C1786G, T1787G, T1796A and TG1796-97AT.

16. A use according to claim 13 the melanoma expresses a mutant RAF kinase.

17. A use according to claim 16 wherein the mutant RAF kinase is a V599E mutation.

## Patentansprüche

1. Verwendung einer Verbindung der Formel (I) 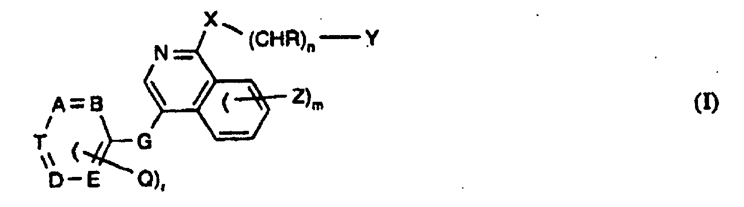 wobei
r gleich 0 bis 2 ist;
n gleich 0 bis 2 ist;
m gleich 0 bis 4 ist;
A, B, D, E und T jeweils unabhängig voneinander für N oder CH stehen, mit der Maßgabe, daß wenigstens einer, aber nicht mehr als drei, der Reste A, B, D, E und T für N stehen;
G für C₁ bis C₇-Alkylen, -CH₂-O-, -CH₂-S-, -CH₂-NH-, -SO₂-, Oxa(-O-), Thia(-S-) oder -NR- oder für ein mit Acyloxy, Oxo, Halogen oder Hydroxy substituiertes C₁₋₇-Alkylen steht;
Q für C₁- bis C₇-Alkyl, vor allem Methyl, steht;
R für H oder C₁- bis C₇-Alkyl steht;
X für Y, -N(R)-, Oxa oder Thio, vorzugsweise -NH-, steht;
Y für H, gegebenenfalls substituiertes C₁- bis C₇-Alkyl, Aryl, Heteroaryl oder gegebenenfalls substituiertes Cycloalkyl steht; und
Z für Amino, ein- oder zweifach substituiertes Amino, Halogen, Alkyl, substituiertes Alkyl, Hydroxy, verethertes oder verestertes Hydorxy, Nitro, Cyano, Carboxy, verestertes Carboxy, Alkanoyl, Carbamoyl, N-einfach oder N,N-zweifach substituiertes Carbamoyl, Amidino, Guanidino, Mercapto, Sulfo, Phenylthio, Phenyl-C₁- bis -C₇-Alkylthio, Alkylphenylthio, Phenylsulfinyl, Phenyl-C₁- bis -C₇-Alkylsulfinyl, Alkylphenylsulfinyl, Phenylsulfonyl, Phenyl-C₁- bis -C₇-Alkansulfonyl oder Alkylphenylsulfonyl steht und wobei, falls mehr als ein Rest Z vorhanden ist (m ≥ 2), die Substituenten Z gleich oder verschieden sind;
oder eines N-Oxids oder eines pharmazeutisch unbedenklichen Salzes davon zur Herstellung eines Arzneimittels für die Behandlung der Krankheit, die durch eine übermäßige Signalgebung über den MAP-Kinase-Signalweg **gekennzeichnet** ist, Melanom, vorausgesetzt, daß es sich bei der Verbindung der Formel (I) nicht um die Verbindung handelt, in der A, B, D, E für CH stehen, T für N, G für Methylen, X für NH, Y für para-CL-Phenyl steht.

2. Verwendung nach Anspruch 1, bei der jeweils
r gleich 0 bis 2 ist;
n gleich 0 oder 1 ist;
m gleich 0 oder 1 ist;
A, B, D und E jeweils für CH stehen und T für N steht oder A, D und E jeweils für CH und B und T für N stehen, oder A, B, E und T für CH stehen und D für N steht, oder A, T, D und E für CH stehen und B für N steht; insbesondere, wobei A, B, D und E jeweils für CH stehen und T für N steht oder A, D und E jeweils für CH und B und T für N stehen.
G für C₁- bis C₇-Alkylen, -CH₂-NH-, -CH₂-O-, Hydroxymethylen oder Benzoyloxymethylen steht;
Q für Methyl steht;
R für H oder C₁- bis C₇-Alkyl steht;
X für -NR-, Oxa oder Thia steht;
Y für Phenyl, das gegebenenfalls mit einem oder zwei gleichen oder verschiedenen Substituenten, ausgewählt aus der Gruppe: Amino; C₁- bis C₇-Alkanoylamino; Halogen; gegebenenfalls substituiertes C₁- bis C₇-Alkyl; Hydroxy; C₁- bis C₇-Alkoxy; Phenyl-C₁- bis -C₇-Alkoxy; Cyano, C₁- bis C₇-Alkenyl, C₈-C₁₂-Alkoxy, C₁- bis C₇-Alkoxycarbonyl, Carbamoyl, C₁- bis C₇-Alkylcarbamoyl, C₁- bis C₇-Alkanoyl, Phenyloxy, Halogen-C₁- bis C₇-alkyloxy, C₁- bis C₇-Alkoxycarbonyl, C₁- bis C₇-Alkylmercapto, Halogen-C₁- bis -C₇-Alkylmercapto, Hydroxy-C₁- bis -C₇-Alkyl, C₁- bis C₇-Alkansulfonyl, Halogen-C₁- bis -C₇-Alkansulfonyl, Phenylsulfonyl und C₁- bis C₇-Alkylendioxy, gebunden an zwei benachbarte Kohlenstoffatome oder wobei zwei benachbarte Stellungen mit Alkylen oder Alkenylen unter Bildung eines 5- bis 7-gliedrigen Rings, der an den Phenylring kondensiert ist, substituiert ist, steht;
Z für Amino; N-C₁- bis C₇-Alkylamino; Hydroxy-C₁- bis C₇-Alkylamino; Phenyl-C₁- bis C₇-alkylamino; N, N-Di-C₁- bis C₇-alkylamino; N-Phenyl-C₁- bis C₇-alkyl-N-C₁- bis C₇-alkylamino; N,N-Di-C₁- bis C₇-alkylphenylamino; C₁- bis C₇-Alkanoylamino; Benzoylamino und Phenyl-C₁- bis C₇-alkoxycarbonylamino steht, wobei der Phenylrest jeweils gegebenenfalls mit Nitro, Amino, Halogen, Amino, N-C₁-bis -C₇-Alkylamino, N,N-Di-C₁- bis -C₇-alkylamino, Hydroxy, Cyano, Carboxy, C₁- bis C₇-Alkoxycarbonyl, C₁-bis C₇-Alkanoyl, Carbamoyl, 2-Hydroxyethylamino, Benzyloxycarbonylamino oder Bromin substituiert ist.

3. Verwendung nach Anspruch 1, bei der jeweils
r gleich 0 ist;
n gleich 0 ist;
m gleich 0 ist;
B, D, E und T für CH stehen und A für N steht oder A, B, D und E jeweils für CH stehen und T für N steht;
G für C₁-₇-Alkylen steht;
X für -NH- steht;
Y für Phenyl steht, das gegebenenfalls mit einem oder zwei gleichen oder verschiedenen Substituenten, ausgewählt aus der Gruppe Halogen und C₁- bis C₇-Alkyl, substituiert ist.

4. Verwendung nach Anspruch 1, bei der jeweils
r gleich 0 ist;
n gleich 0 bis 2 ist;
m gleich 0 ist;
A, B, D und E jeweils für CH stehen und T für N steht;
G für Methylen steht;
R für H steht;
X für -NR steht; und
Y für Phenyl steht, das gegebenenfalls mit Halogen, C₁-bis C₇-Alkyl, C₁- bis C₇-Alkoxy oder gegebenenfalls mit C₁- bis C₇-Alkyl substituiertem Cyclohexyl substituiert ist.

5. Verwendung nach Anspruch 1, bei der jeweils
r gleich 0 ist;
n gleich 0 ist;
m gleich 0 ist;
A, B, D und E jeweils für CH stehen und T für N steht;
G für Methylen steht;
X für -NH- steht; und
Y für Phenyl steht, das mit einem oder zwei gleichen oder verschiedenen Substituenten, ausgewählt aus Halogen und C₁- bis C₇-Alkyl, substituiert ist.

6. Verwendung nach Anspruch 5, bei der Y für Phenyl steht, das in 4-Stellung mit t-Butyl substituiert ist.

7. Verwendung nach Anspruch 5, bei der Y für Phenyl steht, das in 4-Stellung mit Trifluormethyl substituiert ist.

8. Verwendung einer Verbindung des Anspruchs 1, bei der das Melanom mutante RAF-Kinase exprimiert oder eine Wildtyp-RAF-Kinase überexprimiert.

9. Verwendung nach Anspruch 8, bei der jeweils
r gleich 0 bis 2 ist;
n gleich 0 oder 1 ist;
m gleich 0 oder 1 ist;
A, B, D und E jeweils für CH stehen und T für N steht oder A, D und E jeweils für CH und B und T für N stehen, oder A, B, E und T für CH stehen und D für N steht, oder A, T, D und E für CH stehen und B für N steht; insbesondere, wobei A, B, D und E jeweils für CH stehen und T für N steht oder A, D und E jeweils für CH und B und T für N stehen.
G für C₁- bis C₇-Alkylen, -CH₂-NH-, -CH₂-O-, Hydroxymethylen oder Benzoyloxymethylen steht;
Q für Methyl steht;
R für H oder C₁- bis C₇-Alkyl steht;
X für -NR-, Oxa oder Thia steht;
Y für Phenyl, das gegebenenfalls mit einem oder zwei gleichen oder verschiedenen Substituenten, ausgewählt aus der Gruppe: Amino; C₁- bis C₇-Alkanoylamino; Halogen; gegebenenfalls substituiertes C₁- bis C₇-Alkyl; Hydroxy; C₁- bis C₇-Alkoxy; Phenyl-C₁- bis -C₇-Alkoxy; Cyano, C₁- bis C₇-Alkenyl, C₈-C₁₂-Alkoxy, C₁- bis C₇-Alkoxycarbonyl, Carbamoyl, C₁- bis C₇-Alkylcarbamoyl, C₁- bis C₇-Alkanoyl, Phenyloxy, Halogen-C₁- bis C₇-alkyloxy, C₁- bis C₇-Alkoxycarbonyl, C₁- bis C₇-Alkylmercapto, Halogen-C₁- bis -C₇-Alkylmercapto, Hydroxy-C₁- bis -C₇-Alkyl, C₁- bis C₇-Alkansulfonyl, Halogen-C₁- bis -C₇-Alkansulfonyl, Phenylsulfonyl und C₁- bis C₇-Alkylendioxy, gebunden an zwei benachbarte Kohlenstoffatome oder wobei zwei benachbarte Stellungen mit Alkylen oder Alkenylen unter Bildung eines 5- bis 7-gliedrigen Rings, der an den Phenylring kondensiert ist, substituiert ist, steht;
Z für Amino; N-C₁- bis C₇-Alkylamino; Hydroxy-C₁- bis C₇-Alkylamino; Phenyl-C₁- bis C₇-alkylamino; N,N-Di-C₁- bis C₇-alkylamino; N-Phenyl-C₁- bis C₇-alkyl-N-C₁- bis C₇-alkylamino; N,N-Di-C₁- bis C₇-alkylphenylamino; C₁- bis C₇-Alkanoylamino; Benzoylamino und Phenyl-C₁- bis C₇-alkoxycarbonylamino steht, wobei der Phenylrest jeweils gegebenenfalls mit Nitro, Amino, Halogen, Amino, N-C₁-bis -C₇-Alkylamino, N, N-Di-C₁- bis -C₇-alkylamino, Hydroxy, Cyano, Carboxy, C₁- bis C₇-Alkoxycarbonyl, C₁-bis C₇-Alkanoyl, Carbamoyl, 2-Hydroxyethylamino, Benzyloxycarbonylamino oder Bromin substituiert ist.

10. Verwendung nach Anspruch 8, bei der jeweils
r gleich 0 ist;
n gleich 0 ist;
m gleich 0 ist;
B, D, E und T für CH stehen und A für N steht oder A, B, D und E jeweils für CH stehen und T für N steht;
G für C₁₋₇-Alkylen steht;
X für -NH- steht;
Y für Phenyl steht, das gegebenenfalls mit einem oder zwei gleichen oder verschiedenen Substituenten, ausgewählt aus der Gruppe Halogen und C₁- bis C₇-Alkyl, substituiert ist.

11. Verwendung nach Anspruch 8, bei der jeweils
r gleich 0 ist;
n gleich 0 bis 2 ist;
m gleich 0 ist;
A, B, D und E jeweils für CH stehen und T für N steht;
G für Methylen steht;
R für H steht;
X für -NR steht; und
Y für Phenyl steht, das gegebenenfalls mit Halogen, C₁-bis C₇-Alkyl, C₁- bis C₇-Alkoxy oder gegebenenfalls mit niederem Alkyl substituiertem Cyclohexyl substituiert ist.

12. Verwendung nach Anspruch 8, bei der jeweils
r gleich 0 ist;
n gleich 0 ist;
m gleich 0 ist;
A, B, D und E jeweils für CH stehen und T für N steht;
G für Methylen steht;
X für -NH- steht; und
Y für Phenyl steht, das mit einem oder zwei gleichen oder verschiedenen Substituenten, ausgewählt aus Halogen und C₁- bis C₇-Alkyl, substituiert ist.

13. Verwendung nach Anspruch 12, bei der Y für Phenyl steht, das in 4-Stellung mit t-Butyl substituiert ist.

14. Verwendung nach Anspruch 8, bei der jeweils das Melanom eine mutante RAF-Kinase exprimiert.

15. Verwendung nach Anspruch 14, bei der mutante RAF-Kinase einer Mutation im B-RAF-Kinase-Gen, ausgewählt aus G1388A, G1388T, G1394C, G1394A, G1394T, G1403C, G1403A, G1753A, T1782G, G1783C, C1786G, T1787G, T1796A und TG1796-97AT, entspricht.

16. Verwendung nach Anspruch 13, bei der das Melanom eine mutante RAF-Kinase exprimiert.

17. Verwendung nach Anspruch 16, bei der es sich bei der mutanten RAF-Kinase um eine V599E-Mutation handelt.

## Revendications

1. Utilisation d'un composé de formule (I) 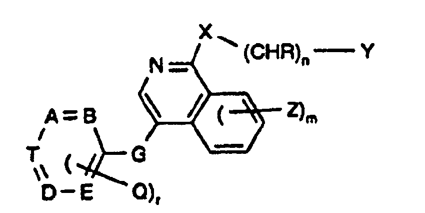 où
r est de 0 à 2 ;
n est de 0 à 2 ;
m est de 0 à 4 ;
A, B, D, E et T sont chacun indépendamment des autres N ou CH, à condition qu'au moins un, mais pas plus de trois, parmi A, B, D, E et T soient N ;
G est un alkylène en C₁ à C₇, -CH₂-O-, -CH₂-S-, -CH₂-NH-, -SO₂-, un oxa (-O-), un thia (-S-) ou -NR-, ou est un alkylène en C₁-C₇ substitué par un acyloxy, un oxo, un halogène ou un hydroxy.
Q est un alkyle en C₁-C₇, en particulier le méthyle ;
R est H ou un alkyle en C₁-C₇ ;
X est Y,-N (R)-, un oxa ou un thio ; de préférence-NH-;
Y est H, un alkyle en C₁-C₇ non substitué ou substitué, un aryle, un hétéroaryle ou un cycloalkyle non substitué ou substitué ; et Z est un amino, un amino mono- ou di-substitué, un halogène, un alkyle, un alkyle substitué, un hydroxy, un hydroxy éthérifié ou estérifié, un nitro, un cyano, un carboxy, un carboxy estérifié, un alcanoyle, un carbamoyle, un carbamoyle N-mono-ou N, N-di-substitué, un amidino, un guanidino, un mercapto, un sulfo, un phénylthio, un phényl(alkyle en C₁-C₇)thio, un alkylphénylthio, un phénylsulfinyle, un phényl(alkyle en C₁-C₇)sulfinyle, un alkylphénylsulfinyle, un phénylsulfonyle, un phényl (alcane en C₁-C₇) sulfonyle ou un alkylphénylsulfonyle, et où, si plus d'un radical Z est présent (m ≥ 2), les substituants Z sont identiques ou différents ;
ou un N-oxyde ou un sel pharmaceutiquement acceptable de celui-ci pour la préparation d'un médicament pour le traitement de la maladie **caractérisée par** une signalisation excessive dans la voie de signalisation de la kinase MAP, le mélanome, à condition que le composé de formule (1) ne soit pas le composé dans lequel A, B, D, E sont CH, T est N, G est le méthylène, X est NH, Y est para - CL - phényle.

2. Utilisation selon la revendication 1 respectivement où
r est de 0 à 2 ;
n est 0 ou 1 ;
m est 0 ou 1 ;
A, B, D et E sont chacun CH et T est N, ou A, D et E sont chacun CH et B et T sont N, ou A, B, E et T sont CH et D est N, ou A, T, D et E sont CH et B est N ; en particulier où A, B, D et E sont chacun CH et T est N, ou A, D et E sont chacun CH et B et T sont N ;
G est un alkylène en C₁-C₇, -CH₂-NH-,-CH₂-O-, l'hydroxyméthylène ou le benzoyloxyméthylène ;
Q est le méthyle ;
R est H ou un alkyle en C₁-C₇;
X est-NR-, un oxa ou un thia ;
Y est un phényle qui est non substitué ou substitué par un ou deux substituants identiques ou différents choisis dans le groupe constitué d'un amino ; un (alcanoyle en C₁-C₇)amino ; un halogène ; un alkyle en C₁-C₇ non substitué ou substitué ; un hydroxy ; un alcoxy en C₁-C₇ ; un phényl(alcoxy en C₁-C₇) ; un cyano, un alcényle en C₁-C₇, un alcoxy en C₈-C₁₂, un (alcoxy en C₁-C₇) carbonyle, un carbamoyle, un (alkyle en C₁-C₇) carbamoyle, un alcanoyle en C₁-C₇, un phényloxy, un halogéno (alkyle en C₁-C₇)oxy, un (alcoxy en C₁-C₇)carbonyle, un (alkyle en C₁-C₇)mercapto, un halogéno(alkyle en C₁-C₇)mercapto, un hydroxy(alkyle en C₁-C₇), un (alcane en C₁-C₇)sulfonyle, un halogéno(alcane en C₁-C₇)sulfonyle, un phénylsulfonyle et un (alkylène en C₁-C₇)dioxy lié à deux atomes de carbone adjacents ou dans lequel deux positions adjacentes sont substituées par un alkylène ou un alcénylène pour former un cycle de 5 à 7 chaînons qui est condensé avec le cycle phényle ;
Z est un amino ; un N-(alkyle en C₁-C₇)amino ; un hydroxy(alkyle en C₁-C₇)amino ; un phényl (alkyle en C₁-C₇)amino ; un N,N-di-(alkyle en C₁-C₇)amino ; un N-phényl(alkyle en C₁-C₇)-N-(alkyle en C₁-C₇)amino ; un N,N-di-(alkyle en C₁-C₇)phénylamino ; un (alcanoyle en C₁-C₇)amino ; un benzoylamino et un phényl (alcoxy en C₁-C₇)carbonylamino, où le radical phényle dans chaque cas est non substitué ou substitué par un nitro, un amino, un halogène, un amino, un N-(alkyle en C₁-C₇)amino, un N,N-di-(alkyle en C₁-C₇)amino, un hydroxy, un cyano, un carboxy, un (alcoxy en C₁-C₇)carbonyle, un alcanoyle en C₁-C₇, un carbamoyle, un 2-hydroxyéthylamino, un benzyloxycarbonylamino ou un brome.

3. Utilisation selon la revendication 1 respectivement où
r est 0 ;
n est 0 ;
m est 0 ;
B, D, E et T sont CH et A est N ou A, B, D et E sont chacun CH et T est N ;
G est un alkylène en C₁-C₇ ;
X est -NH- ;
Y est un phényle qui est non substitué ou substitué par un ou deux substituants identiques ou différents choisis dans le groupe constitué d'un halogène et un alkyle en C₁-C₇.

4. Utilisation selon la revendication 1 respectivement où
r est 0 ;
n est de 0 à 2 ;
m est 0 ;
A, B, D et E sont chacun CH et T est N ;
G est le méthylène ;
R est H ;
X est -NR ; et
Y est un phényle qui est non substitué ou substitué par un halogène, un alkyle en C₁-C₇, un alcoxy en C₁-C₇ ou un cyclohexyle qui est non substitué ou substitué par un alkyle en C₁-C₇.

5. Utilisation selon la revendication 1 respectivement où
r est 0 ;
n est 0 ;
m est 0 ;
A, B, D et E sont chacun CH et T est N ;
G est le méthylène ;
X est -NH- ; et
Y est un phényle qui est substitué par un ou deux substituants identiques ou différents choisis parmi un halogène et un alkyle en C₁-C₇.

6. Utilisation selon la revendication 5 où Y est un phényle qui est substitué à la position 4 par un t-butyle.

7. Utilisation selon la revendication 5 où Y est un phényle qui est substitué à la position 4 par un trifluorométhyle.

8. Utilisation d'un composé de la revendication 1 dans laquelle le mélanome exprime une kinase RAF mutante ou surexprime une kinase RAF de type sauvage.

9. Utilisation selon la revendication 8 respectivement où
r est de 0 à 2 ;
n est 0 ou 1 ;
m est 0 ou 1 ;
A, B, D et E sont chacun CH et T est N, ou A, D et E sont chacun CH et B et T sont N, ou A, B, E et T sont CH et D est N, ou A, T, D et E sont CH et B est N ; en particulier où A, B, D et E sont chacun CH et T est N, ou A, D et E sont chacun CH et B et T sont N ;
G est un alkylène en C₁-C₇, -CH₂-NH-, -CH₂-O-, l'hydroxyméthylène ou le benzoyloxyméthylène ;
Q est le méthyle ;
R est H ou un alkyle en C₁-C₇ ;
X est-NR-, un oxa ou un thia ;
Y est un phényle qui est non substitué ou substitué par un ou deux substituants identiques ou différents choisis dans le groupe constitué d'un amino ; un (alcanoyle en C₁-C₇)amino ; un halogène ; un alkyle en C₁-C₇ non substitué ou substitué ; un hydroxy ; un alcoxy en C₁-C₇ ; un phényl(alcoxy en C₁-C₇) ; un cyano, un alcényle en C₁-C₇, un alcoxy en C₈-C₁₂, un (alcoxy en C₁-C₇) carbonyle, un carbamoyle, un (alkyle en C₁-C₇)carbamoyle, un alcanoyle en C₁-C₇, un phényloxy, un halogéno (alkyle en C₁-C₇)oxy, un (alcoxy en C₁-C₇)carbonyle, un (alkyle en C₁-C₇)mercapto, un halogéno(alkyle en C₁-C₇)mercapto, un hydroxy(alkyle en C₁-C₇), un (alcane en C₁-C₇)sulfonyle, un halogéno(alcane en C₁-C₇)sulfonyle, un phénylsulfonyle et un (alkylène en C₁-C₇)dioxy lié à deux atomes de carbone adjacents ou dans lequel deux positions adjacentes sont substituées par un alkylène ou un alcénylène pour former un cycle de 5 à 7 chaînons qui est condensé avec le cycle phényle ;
Z est un amino ; un N-(alkyle en C₁-C₇)amino ; un hydroxy(alkyle en C₁-C₇)amino ; un phényl (alkyle en C₁-C₇)amino ; un N, N-di- (alkyle en C₁-C₇)amino ; un N-phényl (alkyle en C₁-C₇)-N-(alkyle en C₁-C₇)amino ; un N,N-di-(alkyle en C₁-C₇)phénylamino ; un (alcanoyle en C₁-C₇)amino ; un benzoylamino et un phényl (alcoxy en C₁-C₇)carbonylamino, où le radical phényle dans chaque cas est non substitué ou substitué par un nitro, un amino, un halogène, un amino, un N-(alkyle en C₁-C₇)amino, un N, N-di- (alkyle en C₁-C₇)amino, un hydroxy, un cyano, un carboxy, un (alcoxy en C₁-C₇) carbonyle, un alcanoyle en C₁-C₇, un carbamoyle, un 2-hydroxyéthylamino, un benzyloxycarbonylamino ou un brome.

10. Utilisation selon la revendication 8 respectivement où
r est 0 ;
n est 0 ;
m est 0 ;
B, D, E et T sont CH et A est N ou A, B, D et E sont chacun CH et T est N ;
G est un alkylène en C₁-C₇ ;
X est -NH- ;
Y est un phényle qui est non substitué ou substitué par un ou deux substituants identiques ou différents choisis dans le groupe constitué d'un halogène et un alkyle en C₁-C₇.

11. Utilisation selon la revendication 8 respectivement où
r est 0 ;
n est de 0 à 2 ;
m est 0 ;
A, B, D et E sont chacun CH et T est N ;
G est le méthylène ;
R est H ;
X est -NR ; et
Y est un phényle qui est non substitué ou substitué par un halogène, un alkyle en C₁-C₇, un alcoxy en C₁-C₇ ou un cyclohexyle qui est non substitué ou substitué par un alkyle inférieur.

12. Utilisation selon la revendication 8 respectivement où
r est 0 ;
n est 0 ;
m est 0 ;
A, B, D et E sont chacun CH et T est N ;
G est le méthylène ;
X est -NH- ; et
Y est un phényle qui est substitué par un ou deux substituants identiques ou différents choisis parmi un halogène et un alkyle en C₁-C₇.

13. Utilisation selon la revendication 12 où Y est un phényle qui est substitué à la position 4 par un t-butyle.

14. Utilisation selon la revendication 8 respectivement où le mélanome exprime une kinase RAF mutante.

15. Utilisation selon la revendication 14 où la kinase RAF mutante correspond à une mutation dans le gène de kinase B-RAF choisie parmi G1388A, G1388T, G1394C, G1394A, G1394T, G1403C, G1403A, G1753A, T1782G, G1783C, C1786G, T1787G, T1796A et TG 1796-97AT.

16. Utilisation selon la revendication 13 où le mélanome exprime une kinase RAF mutante.

17. Utilisation selon la revendication 16 où la kinase RAF mutante est une mutation V599E.


### SEQUENCE LISTING

<110> Novartis AG
<120> Method of Treatment
<130> 4-32905A
<160> 28
<170> Patent In version 3.2
<210> 1
   <211> 20
   <212> DNA
   <213> Homo sapiens
<400> 1
   acagtgggac aaagaattga 20
<210> 2
   <211> 20
   <212> DNA
   <213> Homo sapiens
<400> 2
   acagtgggac aaagaattgt 20
<210> 3
   <211> 20
   <212> DNA
   <213> Homo sapiens
<400> 3
   ggacaaagaa ttggatctgc 20
<210> 4
   <211> 20
   <212> DNA
   <213> Homo sapiens
<400> 4
   ggacaaagaa ttggatctga 20
<210> 5
   <211> 20
   <212> DNA
   <213> Homo sapiens
<400> 5
   ggacaaagaa ttggatctgt 20
<210> 6
   <211> 20
   <212> DNA
   <213> Homo sapiens
<400> 6
   attggatctg gatcatttgc 20
<210> 7
   <211> 20
   <212> DNA
   <213> Homo sapiens
<400> 7
   attggatctg gatcatttga 20
<210> 8
   <211> 23
   <212> DNA
   <213> Homo sapiens
<400> 8
   gagtaataat atatttcttc ata 23
<210> 9
   <211> 20
   <212> DNA
   <213> Homo sapiens
<400> 9
   cagtaaaaat aggtgatttg 20
<210> 10
   <211> 21
   <212> DNA
   <213> Homo sapiens
<400> 10
   cagtaaaaat aggtgatttt c 21
<210> 11
   <211> 22
   <212> DNA
   <213> Homo sapiens
<400> 11
   gtaaaaatag gtgattttgg tg 22
<210> 12
   <211> 23
   <212> DNA
   <213> Homo sapiens
<400> 12
   gtaaaaatag gtgattttgg tcg 23
<210> 13
   <211> 20
   <212> DNA
   <213> Homo sapiens
<400> 13
   gattttggtc tagctacaga 20
<210> 14
   <211> 21
   <212> DNA
   <213> Homo sapiens
<400> 14
   gattttggtc tagctacaga t 21
<210> 15
   <211> 23
   <212> DNA
   <213> Homo sapiens
<400> 15 23
   tgtcaccaca ttacatactt acc 23
<210> 16
   <211> 23
   <212> DNA
   <213> Homo sapiens
<400> 16 23
   tgtcaccaca ttacatactt acc 23
<210> 17
   <211> 23
   <212> DNA
   <213> Homo sapiens
<400> 17 23
   tgtcaccaca ttacatactt acc 23
<210> 18
   <211> 23
   <212> DNA
   <213> Homo sapiens
<400> 18 23
   tgtcaccaca ttacatactt acc 23
<210> 19
   <211> 23
   <212> DNA
   <213> Homo sapiens
<400> 19 23
   tgtcaccaca ttacatactt acc 23
<210> 20
   <211> 23
   <212> DNA
   <213> Homo sapiens
<400> 20 23
   tgtcaccaca ttacatactt acc 23
<210> 21
   <211> 23
   <212> DNA
   <213> Homo sapiens
<400> 21 23
   tgtcaccaca ttacatactt acc 23
<210> 22
   <211> 21
   <212> DNA
   <213> Homo sapiens
<400> 22
   gactttctag taactcagca g 21
<210> 23
   <211> 21
   <212> DNA
   <213> Homo sapiens
<400> 23 21
   gactttctag taactcagca g 21
<210> 24
   <211> 21
   <212> DNA
   <213> Homo sapiens
<400> 24 21
   gactttctag taactcagca g 21
<210> 25
   <211> 21
   <212> DNA
   <213> Homo sapiens
<400> 25 21
   gactttctag taactcagca g 21
<210> 26
   <211> 21
   <212> DNA
   <213> Homo sapiens
<400> 26 21
   gactttctag taactcagca g 21
<210> 27
   <211> 21
   <212> DNA
   <213> Homo sapiens
<400> 27 21
   gactttctag taactcagca g 21
<210> 28
   <211> 21
   <212> DNA
   <213> Homo sapiens
<400> 28
   gactttctag taactcagca g 21